# EUROPEAN PATENT APPLICATION

(11) **EP 1 602 363 A1**
(43) Date of publication of application: **07.12.2005**
(21) Application number: 05253398.1
(22) Date of filing: 02.06.2005
(51) Int. Cl.: A61K 9/44

(54) **Immediate release dosage form comprising shell having openings therein**

(30) Priority: 04.06.2004 US 860972
(71) Applicant: McNeil-PPC, Inc., Skillman, NJ 08558 (US)
(72) Inventor: Sowden, Harry S., Glenside, PA 19038 (US); McNally, Gerard P., Berwyn, PA 19312 (US)
(74) Representative: Tunstall, Christopher Stephen

(57) **Abstract**

The invention provides an immediate release dosage form having a solid core and a shell readily soluble in gastrointestinal fluids. The dosage form also comprises one or more openings in the shell.

## Description

### CROSS-REFERENCE TO RELATED APPLICATIONS

This is a continuation-in-part of copending application 10/393,610, filed March 21, 2003, which is a continuation-in-part of PCT Application Nos. PCT/US02/31129, filed September 28, 2002; PCT/US02/31117, filed September 28, 2002; PCT/US02/31062, filed September 28, 2002; PCT/US02/31024, filed September 28, 2002; and PCT/US02/31163, filed September 28, 2002, which are each continuations-in-part of USSN 09/966,939, filed September 28, 2001; USSN 09/966,509, filed September 28, 2001; USSN 09/966,497, filed September 28, 2001; USSN 09/967,414, filed September 28, 2001; and USSN 09/966,450, filed September 28, the disclosures of all of the above which is incorporated herein by reference in its entirety.

### Field of the Invention

This invention relates to a dosage form providing immediate release of an active ingredient. The dosage form comprises a solid core and a shell surrounding the core. The shell has one or more openings therein.

### Background of the Invention

Certain dosage forms containing apertures or embossments are known. For instance, "osmotic pump" dosage forms for the administration of pharmaceutically active ingredients are known in the art. They typically comprise a semi-permeable wall that surrounds a reservoir that contains drug. The wall is permeable to the passage of an external fluid, impermeable to the passage of drug, and has a passageway through the semi-permeable wall for delivering drug from the osmotic system. For example, U.S. Patent No. 4,576,604 discloses an osmotic device comprising a drug compartment surrounded by a wall (coating) having a passageway therein. The wall may comprise an immediate release dose of drug, and the inner drug compartment may comprise a sustained release dose of drug.

U.S. Patent No. 4,449,983 discloses another osmotic device comprising two separately housed drugs that are separately dispensed from the device. The device comprises two compartments, one for each drug, separated by a partition. Each compartment has an orifice for communicating with the exterior of the device.

U.S. Patent No. 3,823,816 discloses a water-soluble package provided in the form of a hard shell capsule filled with powder, granules, or the like. The capsule is apertured, and a water-soluble barrier film covers the apertures. The film is more water soluble than the capsule so that when the package contacts water, the film rather than the capsule dissolves first, and consequently exposing the contents for dissolution and/or release by way of the apertures while the capsule is intact.

U.S. Patent No. 5,256,440 relates to an intagliated dosage form comprising one or more circumscribed regions on its surface. The dosage form is spray coated with a latex polymer. When placed in an environment of use, the latex coating within the circumscribed region is reproducibly expelled, leaving a coated core tablet with a predefined aperture, which exposes a discrete portion of the core surface to the environment of use.

One known method of producing gelatin coated dosage forms is via an enrobing process wherein two separate films made of gelatinous material are applied to opposite sides of a tablet by a pair of rotary dies, as disclosed for example, in U.S. Patent Nos. 5,146,730 and 5,459,983. Film formulations for producing gelcaps and geltabs prepared via enrobing methods such as those disclosed in U.S. Patent Nos. 5,146,730 and 5,459,983 typically comprises a water-based gelatin preparation having about 45% gelatin and about 9% plasticizer (glycerin and/or sorbitol) by weight. Glycerin and sorbitol can be used as single plasticizers or in combination with each other. In addition, other sugars and poly-hydroxy compounds can be used as additives and plasticizers. If a tamper-evident gelatin-coated medicine tablet is the desired end product, then the ratio of plasticizer to gelatin in the gelatin formulation should be in the range of about 1:5.

Another conventional method for forming a shell (or coating), on a core (or substrate), is that disclosed in WO 01/57144 which utilizes the principles of electrostatic deposition to form the coating. At least one of the core or the shell preferably incorporates one or more "charge control agents," such as metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts, in an amount from about 1% to about 10% by weight of the shell.

Applicants have now discovered that an immediate release dosage form may be made from a solid core and a shell surrounding the core, wherein the core has a density of at least about 0.9 g/cc and a percent porosity of less than 40%, preferably less than 35%, most preferably 30%. The shell comprises one or more openings therein and is readily soluble in gastrointestinal fluids. The shell is preferably applied to the core by enrobing.

### Summary of the Invention

The present invention provides a dosage form containing at least one active ingredient, which comprises a core and a shell surrounding the core, wherein the core has a density of at least about 0.9 g/cc and a percent porosity of less than 40%, preferably less than 35%, most preferably 30%, the shell comprises one or more openings, the shell is readily soluble in gastrointestinal fluids, and the dosage form provides for immediate release of at least one active ingredient upon contact of the dosage form with a liquid medium.

The invention also provides a dosage form comprising a core having an outer surface and a shell having outer and inner surfaces, wherein at least a portion of the shell surrounds the core such that the shell inner surface resides substantially conformally upon the core outer surface, at least a portion of the shell comprises one or more openings therein, one or more of the openings are from about 200 to about 2000 microns in diameter or width, at least a portion of the shell is readily soluble in gastrointestinal fluids, the average shell thickness is in the range from about 100 to about 400 microns, the shell comprises less than about 50% crystallizable sugar, and the dosage form is substantially free of charge control agents and the dosage form provides for immediate release of at least one active ingredient upon contact of the dosage form with a liquid medium.

### Brief Description of the Drawings

Figures 1-5 depict dosage forms according to the invention.
Figure 6 depicts one embodiment having openings according to the invention.
Figure 7 depicts a method of producing a dosage form according to the invention using an enrobing process.
Figure 8 depicts another method of producing a dosage form according to the invention using an enrobing process.
Figure 9 depicts a further method of producing a dosage form according to the invention using an enrobing process.
Figure 10 depicts a method of producing a dosage form according to the invention using an enrobing process with a capsule comprising openings.
Figure 11 depicts a method of producing a dosage form according to the invention using a dipping process.
Figure 12 depicts another method of producing a dosage form according to the invention using a dipping process.
Figure 13 depicts a method of producing a dosage form according to the invention using a transfer process.
Figure 14 depicts a method of producing openings in a shell of a dosage form according to the invention using grinding means.
Figures 15a and 15b depict one method of producing openings in a shell of a dosage form according to the invention using hot pins.
Figures 16a and 16b depict one method of producing openings in a shell of a dosage form according to the invention using punching means.
Figures 17a and 17b depict a method of producing openings in a shell of a dosage form according to the invention by injection molding.

### Detailed Description of the Invention

As used herein, the term "dosage form" applies to any solid object, semi-solid, or liquid composition designed to contain a specific pre-determined amount (dose) of a certain ingredient, for example an active ingredient as defined below. Suitable dosage forms may be pharmaceutical drug delivery systems, including those for oral administration, buccal administration, rectal administration, topical or mucosal delivery, or subcutaneous implants, or other implanted drug delivery systems; or compositions for delivering minerals, vitamins and other nutraceuticals, oral care agents, flavorants, and the like. Preferably the dosage forms of the present invention are considered to be solid, however they may contain liquid or semi-solid components. In a particularly preferred embodiment, the dosage form is an orally administered system for delivering a pharmaceutical active ingredient to the gastro-intestinal tract of a human.

Suitable active ingredients for use in this invention include for example pharmaceuticals, minerals, vitamins and other nutraceuticals; oral care agents, flavorants and mixtures thereof. Suitable pharmaceuticals include analgesics, anti-inflammatory agents, antiarthritics, anesthetics, antihistamines, antitussives, antibiotics, anti-infective agents, antivirals, anticoagulants, antidepressants, antidiabetic agents, antiemetics, antiflatulents, antifungals, antispasmodics, appetite suppressants, bronchodilators, cardiovascular agents, central nervous system agents, central nervous system stimulants, decongestants, oral contraceptives, diuretics, expectorants, gastrointestinal agents, migraine preparations, motion sickness products, mucolytics, muscle relaxants, osteoporosis preparations, polydimethylsiloxanes, respiratory agents, sleep-aids, urinary tract agents and mixtures thereof.

Suitable oral care agents include breath fresheners, tooth whiteners, antimicrobial agents, tooth mineralizers, tooth decay inhibitors, topical anesthetics, mucoprotectants, and the like.

Suitable flavorants include menthol, peppermint, mint flavors, fruit flavors, chocolate, vanilla, bubblegum flavors, coffee flavors, liqueur flavors and combinations and the like.

Examples of suitable gastrointestinal agents include antacids such as calcium carbonate, magnesium hydroxide, magnesium oxide, magnesium carbonate, aluminum hydroxide, sodium bicarbonate, dihydroxyaluminum sodium carbonate; stimulant laxatives, such as bisacodyl, cascara sagrada, danthron, senna, phenolphthalein, aloe, castor oil, ricinoleic acid, and dehydrocholic acid, and mixtures thereof; H2 receptor antagonists, such as famotadine, ranitidine, cimetadine, nizatidine; proton pump inhibitors such as omeprazole or lansoprazole; gastrointestinal cytoprotectives, such as sucraflate and misoprostol; gastrointestinal prokinetics, such as prucalopride, antibiotics for H. pylori, such as clarithromycin, amoxicillin, tetracycline, and metronidazole; antidiarrheals, such as diphenoxylate and loperamide; glycopyrrolate; antiemetics, such as ondansetron, analgesics, such as mesalamine.

In one embodiment of the invention, the active ingredient may be selected from bisacodyl, famotadine, ranitidine, cimetidine, prucalopride, diphenoxylate, loperamide, lactase, mesalamine, bismuth, antacids, and pharmaceutically acceptable salts, esters, isomers, and mixtures thereof.

In another embodiment, the active ingredient is selected from analgesics, antiinflammatories, and antipyretics, e.g. non-steroidal anti-inflammatory drugs (NSAIDs), including propionic acid derivatives, e.g. ibuprofen, naproxen, ketoprofen and the like; acetic acid derivatives, e.g. indomethacin, diclofenac, sulindac, tolmetin, and the like; fenamic acid derivatives, e.g. mefanamic acid, meclofenamic acid, flufenamic acid, and the like; biphenylcarbodylic acid derivatives, e.g. diflunisal, flufenisal, and the like; and oxicams, e.g. piroxicam, sudoxicam, isoxicam, meloxicam, and the like. In one particular embodiment, the active ingredient is selected from propionic acid derivative NSAID, e.g. ibuprofen, naproxen, flurbiprofen, fenbufen, fenoprofen, indoprofen, ketoprofen, fluprofen, pirprofen, carprofen, oxaprozin, pranoprofen, suprofen, and pharmaceutically acceptable salts, derivatives, and combinations thereof. In another particular embodiment of the invention, the active ingredient may be selected from acetaminophen, acetyl salicylic acid, ibuprofen, naproxen, ketoprofen, flurbiprofen, diclofenac, cyclobenzaprine, meloxicam, rofecoxib, celecoxib, and pharmaceutically acceptable salts, esters, isomers, and mixtures thereof.

In another embodiment of the invention, the active ingredient may be selected from pseudoephedrine, phenylpropanolamine, chlorpheniramine, dextromethorphan, diphenhydramine, astemizole, terfenadine, fexofenadine, loratadine, desloratadine, cetirizine, mixtures thereof and pharmaceutically acceptable salts, esters, isomers, and mixtures thereof.

Examples of suitable polydimethylsiloxanes, which include, but are not limited to dimethicone and simethicone, are those disclosed in United States Patent Nos. 4,906,478, 5,275,822, and 6,103,260, the contents of each is expressly incorporated herein by reference. As used herein, the term "simethicone" refers to the broader class of polydimethylsiloxanes, including but not limited to simethicone and dimethicone.

The active ingredient or ingredients are present in the dosage form in a therapeutically effective amount, which is an amount that produces the desired therapeutic response upon oral administration and can be readily determined by one skilled in the art. In determining such amounts, the particular active ingredient being administered, the bioavailability characteristics of the active ingredient, the dosing regimen, the age and weight of the patient, and other factors must be considered, as known in the art. Typically, the dosage form comprises at least about 1 weight percent, preferably, the dosage form comprises at least about 5 weight percent, e.g. at least about 25 weight percent of a combination of one or more active ingredients. In one preferred embodiment, a core comprises a total of at least about 50 weight percent, e.g. at least about 70 weight percent, say at least about 80 weight percent (based on the weight of the core) of one or more active ingredients.

The active ingredient or ingredients may be present in the dosage form in any form. For example, the active ingredient may be dispersed at the molecular level, e.g. melted or dissolved, within the dosage form, or may be in the form of particles, which in turn may be coated or uncoated. If the active ingredient is in form of particles, the particles (whether coated or uncoated) typically have an average particle size of about 1-2000 microns. In one preferred embodiment, such particles are crystals having an average particle size of about 1-300 microns. In another preferred embodiment, the particles are granules or pellets having an average particle size of about 50-2000 microns, preferably about 50-1000 microns, most preferably about 100-800 microns.

The core may be any solid form. The core may be prepared by any suitable method, including for example compression or molding. As used herein, "core" refers to a material that is at least partially enveloped or surrounded by another material. Preferably, the core is a self-contained unitary object, such as a tablet or capsule. Typically, the core comprises a solid, for example, the core may be a compressed or molded tablet, hard or soft capsule, suppository, or a confectionery form such as a lozenge, nougat, caramel, fondant, or fat based composition. In certain other embodiments, the core or a portion thereof may be in the form of a semi-solid or a liquid in the finished dosage form. For example the core may comprise a liquid filled capsule, or a semisolid fondant material. In embodiments in which the core comprises a flowable component, such as a plurality of granules or particles, or a liquid, the core preferably additionally comprises an enveloping component, such as a capsule shell, or a coating, for containing the flowable material. In certain particular embodiments in which the core comprises an enveloping component, the shell or shell portions of the present invention are in direct contact with the enveloping component of the core, which separates the shell from the flowable component of the core.

In one embodiment the core is a compressed tablet having a hardness from about 2 to about 30 kp/cm², e.g. from about 6 to about 25 kp/cm². "Hardness" is a term used in the art to describe the diametric breaking strength of either the core or the coated solid dosage form as measured by conventional pharmaceutical hardness testing equipment, such as a Schleuniger Hardness Tester. In order to compare values across different size tablets, the breaking strength must be normalized for the area of the break. This normalized value, expressed in kp/cm², is sometimes referred in the art as tablet tensile strength. A general discussion of tablet hardness testing is found in Leiberman et al., *Pharmaceutical Dosage Forms - Tablets,* Volume 2, 2^{nd} ed., Marcel Dekker Inc., 1990, pp. 213 - 217, 327 - 329.

The core may have one of a variety of different shapes. For example, the core may be shaped as a polyhedron, such as a cube, pyramid, prism, or the like; or may have the geometry of a space figure with some non-flat faces, such as a cone, truncated cone, cylinder, sphere, torus, or the like. In certain embodiments, a core has one or more major faces. For example, in embodiments wherein a core is a compressed tablet, the core surface typically has two opposing major faces formed by contact with the upper and lower punch faces in the compression machine. In such embodiments the core surface typically further comprises a "belly-band" located between the two major faces, and formed by contact with the die walls in the compression machine. A core may also comprise a multilayer tablet.

Exemplary core shapes that may be employed include tablet shapes formed from compression tooling shapes described by "The Elizabeth Companies Tablet Design Training Manual" (Elizabeth Carbide Die Co., Inc., p. 7 (McKeesport, Pa.) (incorporated herein by reference) as follows (the tablet shape corresponds inversely to the shape of the compression tooling):
1. Shallow Concave.
2. Standard Concave.
3. Deep Concave.
4. Extra Deep Concave.
5. Modified Ball Concave.
6. Standard Concave Bisect.
7. Standard Concave Double Bisect.
8. Standard Concave European Bisect.
9. Standard Concave Partial Bisect.
10. Double Radius.
11. Bevel & Concave.
12. Flat Plain.
13. Flat-Faced-Beveled Edge (F.F.B.E.).
14. F.F.B.E. Bisect.
15. F.F.B.E. Double Bisect.
16. Ring.
17. Dimple.
18. Ellipse.
19. Oval.
20. Capsule.
21. Rectangle.
22. Square.
23. Triangle.
24. Hexagon.
25. Pentagon.
26. Octagon.
27. Diamond.
28. Arrowhead.
29. Bullet.
30. Shallow Concave.
31. Standard Concave.
32. Deep Concave.
33. Extra Deep Concave.
34. Modified Ball Concave.
35. Standard Concave Bisect.
36. Standard Concave Double Bisect.
37. Standard Concave European Bisect.
38. Standard Concave Partial Bisect.
39. Double Radius.
40. Bevel & Concave.
41. Flat Plain.
42. Flat-Faced-Beveled Edge (F.F.B.E.).
43. F.F.B.E. Bisect.
44. F.F.B.E. Double Bisect.
45. Ring.
46. Dimple.
47. Ellipse.
48. Oval.
49. Capsule.
50. Rectangle.
51. Square.
52. Triangle.
53. Hexagon.
54. Pentagon.
55. Octagon.
56. Diamond.
57. Arrowhead.
58. Bullet.
59. Barrel.
60. Half Moon.
61. Shield.
62. Heart.
63. Almond.
64. House/Home Plate.
65. Parallelogram.
66. Trapezoid.

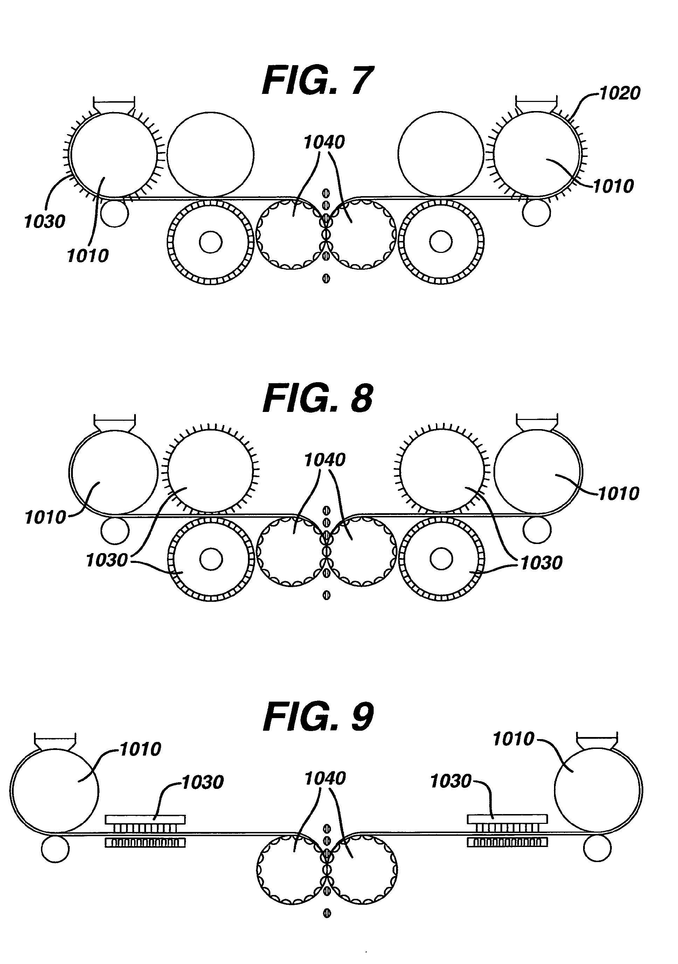

67. Figure 8/Bar Bell.
68. Bow Tie.
69. Uneven Triangle.

The core typically comprises active ingredient and a variety of excipients, depending on the method by which it is made.

In embodiments in which the core is made by compression, suitable excipients include fillers, binders, disintegrants, lubricants, glidants, and the like, as known in the art. In embodiments in which the core is made by compression and additionally confers modified release of an active ingredient contained therein, such core preferably further comprises a release-modifying compressible excipient.

Suitable fillers for use in making the core by compression include water-soluble compressible carbohydrates such as sugars, which include dextrose, sucrose, maltose, and lactose, sugar-alcohols, which include mannitol, sorbitol, maltitol, xylitol, starch hydrolysates, which include dextrins, and maltodextrins, and the like, water insoluble plastically deforming materials such as microcrystalline cellulose or other cellulosic derivatives, water-insoluble brittle fracture materials such as dicalcium phosphate, tricalcium phosphate and the like and mixtures thereof.

Suitable binders for making the core by compression include dry binders such as polyvinyl pyrrolidone, hydroxypropylmethylcellulose, and the like; wet binders such as water-soluble polymers, including hydrocolloids such as acacia, alginates, agar, guar gum, locust bean, carrageenan, carboxymethylcellulose, tara, gum arabic, tragacanth, pectin, xanthan, gellan, gelatin, maltodextrin, galactomannan, pusstulan, laminarin, scleroglucan, inulin, whelan, rhamsan, zooglan, methylan, chitin, cyclodextrin, chitosan, polyvinyl pyrrolidone, cellulosics, sucrose, starches, and the like; and derivatives and mixtures thereof.

Suitable disintegrants for making the core by compression, include sodium starch glycolate, cross-linked polyvinylpyrrolidone, cross-linked carboxymethylcellulose, starches, microcrystalline cellulose, and the like.

Suitable lubricants for making the core by compression include long chain fatty acids and their salts, such as magnesium stearate and stearic acid, talc, glycerides and waxes.

Suitable glidants for making the core by compression, include colloidal silicon dioxide, and the like.

In certain embodiments, the core or a portion thereof may optionally comprise release modifying excipients as known in the art, for example as disclosed in commonly assigned, copending U.S. Application Serial No. 10/432488, filed September 28, 2002, the disclosure of which is incorporated by reference herein. Suitable release-modifying compressible excipients for making the core by compression include swellable erodible hydrophilic materials, insoluble edible materials, pH-dependent polymers, and the like.

Suitable pharmaceutically acceptable adjuvants for making the cores by compression include, preservatives; high intensity sweeteners such as aspartame, acesulfame potassium, sucralose, and saccharin; flavorants; colorants; antioxidants; surfactants; wetting agents; and the like and mixtures thereof.

In embodiments wherein one or more cores are prepared by compression, a dry blending (i.e. direct compression), or wet granulation process may be employed, as known in the art. In a dry blending (direct compression) method, the active ingredient or ingredients, together with the excipients, are blended in a suitable blender, than transferred directly to a compression machine for pressing into tablets. In a wet granulation method, the active ingredient or ingredients, appropriate excipients, and a solution or dispersion of a wet binder (e.g. an aqueous cooked starch paste, or solution of polyvinyl pyrrolidone) are mixed and granulated. Alternatively a dry binder may be included among the excipients, and the mixture may be granulated with water or other suitable solvent. Suitable apparatuses for wet granulation are known in the art, including low shear, e.g. planetary mixers; high shear mixers; and fluid beds, including rotary fluid beds. The resulting granulated material is dried, and optionally dry-blended with further ingredients, e.g. adjuvants and/or excipients such as for example lubricants, colorants, and the like. The final dry blend is then suitable for compression. Methods for direct compression and wet granulation processes are known in the art, and are described in detail in, for example, Lachman, et al., The Theory and Practice of Industrial Pharmacy, Chapter 11 (3rd ed. 1986).

The dry-blended, or wet granulated, powder mixture is typically compacted into tablets using a rotary compression machine as known in the art, such as for example those commercially available from Fette America Inc., Rockaway, NJ, or Manesty Machines LTD, Liverpool, UK. In a rotary compression machine, a metered volume of powder is filled into a die cavity, which rotates as part of a "die table" from the filling position to a compaction position where the powder is compacted between an upper and a lower punch to an ejection position where the resulting tablet is pushed from the die cavity by the lower punch and guided to an ejection chute by a stationary "take-off" bar.

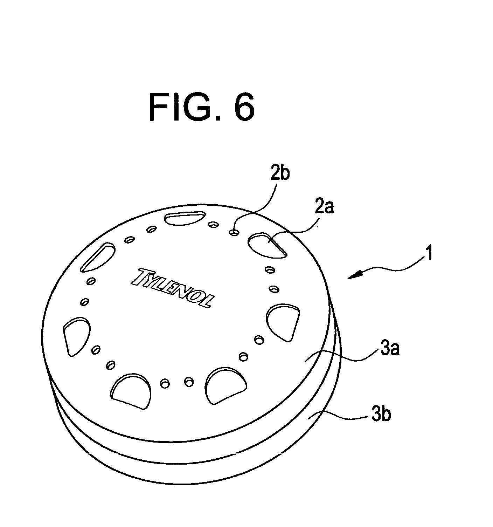

In one optional embodiment, the core may be prepared by the compression methods and apparatus described in copending U.S. Patent Application Serial No. 09/966,509, pages 16-27, the disclosure of which is incorporated herein by reference. Specifically, the core is made using a rotary compression module comprising a fill zone, insertion zone, compression zone, ejection zone, and purge zone in a single apparatus having a double row die construction as shown in Figure 6 of U.S. patent application Serial No. 09/966,509. The dies of the compression module are preferably filled using the assistance of a vacuum, with filters located in or near each die.

Cores made by compression may be single or multi-layer, for example bilayer, tablets.

The cores have a density of at least about 0.9 g/cc, e.g. at least about 1.0 g/cc and a percent porosity of less than 40%, preferably less than 35%, most preferably 30%. Porosity of a powder is a ratio of void volume to bulk volume. The void volume is the volume of spaces between the particles, while the bulk volume is the total, space occupied. Percent porosity is that ratio expressed as a percentage. These values can be measured using a mercury intrusion porosimeter, such as the Autopore IV 9500 V1.05, available from Micrometics Corporation, at a mercury filling pressure of 1.32 to 1.33 psia, mercury contact angle of 130 degrees, and surface tension 485 dynes/cm. Exemplary cores include a 385 mg compressed soft tablet with a volume of 0.4 cubic centimeters, and a 586 mg compressed tablet with a volume of about 0.5 cc.

A shell surrounds the cores. The shell comprises one or more openings therein. The opening or openings provide a passageway for communication between the core and the exterior of the dosage form. The openings may extend completely through the thickness of the shell to contact the core, or only partially through the shell.

The shell may be substantially unitary and continuous with the exception of the openings therein, or the shell may comprise multiple portions, e.g. a first shell portion and a second shell portion. In certain embodiments the shell or shell portions are in direct contact with the core. In certain other embodiments, the shell or shell portions are in direct contact with a subcoating, which substantially surrounds the core. In embodiments in which the shell comprises a first and second shell portion, at least a first shell portion comprises openings therein.

In certain embodiments the first shell portion and second shell portion are compositionally different. As used herein, the term "compositionally different" means having features that are readily distinguishable by qualitative or quantitative chemical analysis, physical testing, or visual observation. For example, the first and second shell portions may contain different ingredients, or different levels of the same ingredients, or the first and second shell portions may have different physical or chemical properties, different functional properties, or be visually distinct. Examples of physical or chemical properties that may be different include hydrophilicity, hydrophobicity, hygroscopicity, elasticity, plasticity, tensile strength, crystallinity, and density. Examples of functional properties which may be different include rate and/or extent of dissolution of the material itself or of an active ingredient therefrom, rate of disintegration of the material, permeability to active ingredients, permeability to water or aqueous media, and the like. Examples of visual distinctions include size, shape, topography, or other geometric features, color, hue, opacity, and gloss.

In one embodiment, the dosage form of the invention comprises: a) a core containing an active ingredient; b) an optional subcoating that substantially covers the core; and c) a shell comprising first and second shell portions residing on the surface of the subcoating, the first shell portion comprising one or more openings, and the first shell portion being readily soluble in gastrointestinal fluids. As used herein, "substantially covers" shall mean at least about 95 percent of the surface area of the core is covered by the subcoating. The subcoating can optionally contain colorants, such as dyes, pigments and mixtures thereof, that produce opaque, pearlescent or translucent effects.

The use of subcoatings is well known in the art and disclosed in, for example, United States Patent Nos. 3,185,626, which is incorporated by reference herein. Any composition suitable for film-coating a tablet may be used as a subcoating according to the present invention. Examples of suitable subcoatings are disclosed in United States Patent Nos. 4,683,256, 4,543,370, 4,643,894, 4,828,841, 4,725,441, 4,802,924, 5,630,871, and 6,274,162, which are all incorporated by reference herein. Additional suitable subcoatings include one or more of the following ingredients: cellulose ethers such as hydroxypropylmethylcellulose, hydroxypropylcellulose, and hydroxyethylcellulose; polycarbohydrates such as xanthan gum, starch, and maltodextrin; plasticizers including for example, glycerin, polyethylene glycol, propylene glycol, dibutyl sebecate, triethyl citrate, vegetable oils such as castor oil, surfactants such as Polysorbate-80, sodium lauryl sulfate and dioctyl-sodium sulfosuccinate; polycarbohydrates, pigments, and opacifiers.

In one embodiment, the subcoating comprises from about 2 percent to about 8 percent, e.g. from about 4 percent to about 6 percent of a water-soluble cellulose ether and from about 0.1 percent to about 1 percent, castor oil, as disclosed in detail in United States Patent No. 5,658,589, which is incorporated by reference herein. In another embodiment, the subcoating comprises from about 20 percent to about 50 percent, e.g., from about 25 percent to about 40 percent of HPMC; from about 45 percent to about 75 percent, e.g., from about 50 percent to about 70 percent of maltodextrin; and from about 1 percent to about 10 percent, e.g., from about 5 percent to about 10 percent of PEG 400.

The dried subcoating typically is present in an amount, based upon the dry weight of the core, from about 0 percent to about 5 percent.

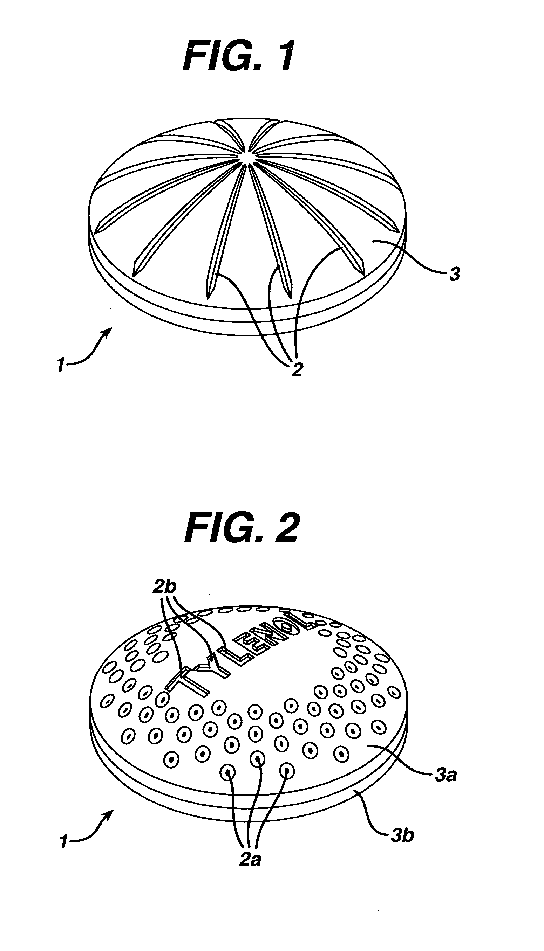

Figure 1 depicts a dosage form 1 according to the invention comprising a shell 3 having a plurality of openings 2. Openings 2 are shaped as elongated slits, and do not extend all the way through the shell 3 to the core (not shown) under the shell 3.

Figure 2 depicts another dosage form according to the invention. The dosage form 1 comprises a core (not shown) covered a shell made of a first shell portion 3a and a second shell portion 3b. Shell portion 3a contains a plurality of openings 2a, 2b. Openings 2a are in the shape of dimples, while openings 2b are in the shape of letters.

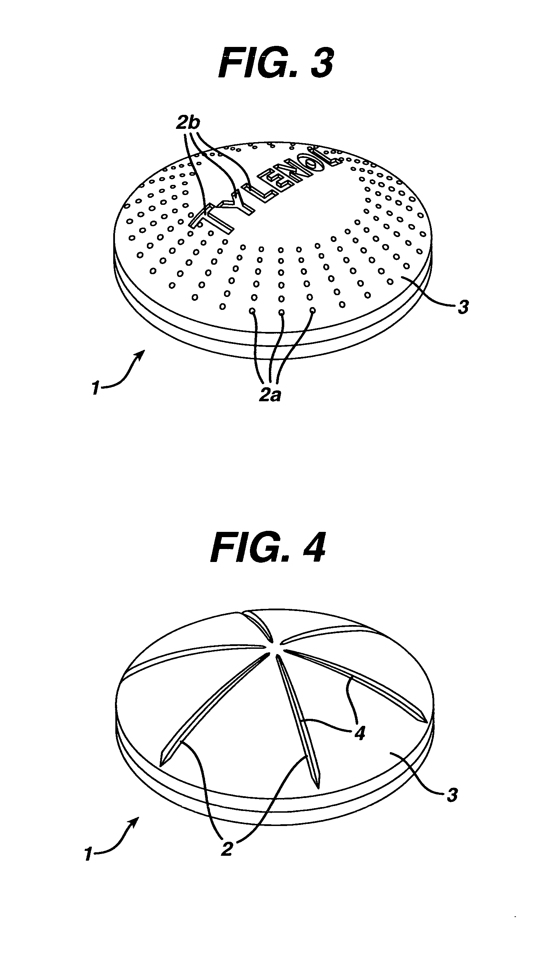

Figure 3 illustrates another dosage form according to the invention. Dosage form 1 comprises a core (not shown) covered by a shell 3, which comprises openings 2a, 2b. Openings 2a are in the shape of circular holes, while openings 2b are in the shape of letters. Openings 2a preferably pass entirely through shell 3 and thereby expose a portion of the subcoated or uncoated core.

Figure 4 shows another dosage form according to the invention. Dosage form 1 comprises a core 4 surrounded by a shell 3 having openings 2. Core 4 is partially visible at the base of each opening 2 in shell 3.

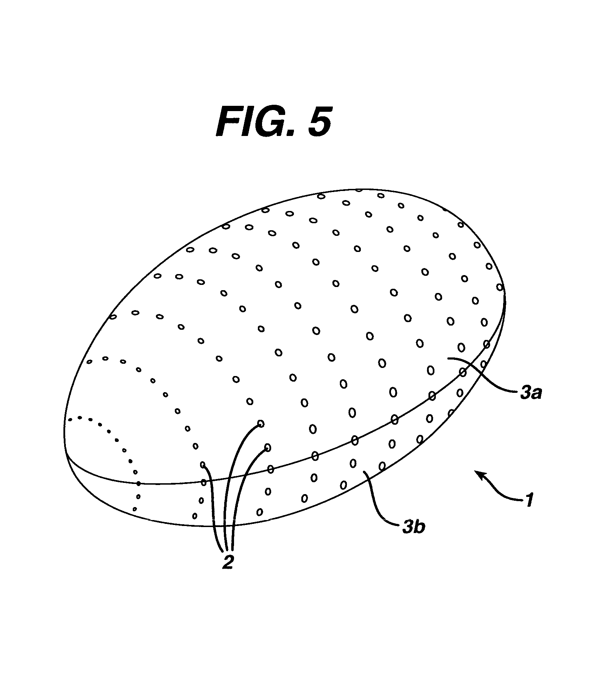

Figure 5 depicts a further dosage form according to the invention. Dosage form 1 comprises a football-shaped core (not shown) covered by a shell comprising a first shell portion 3a and a second shell portion 3b. First shell portion 3a comprises a plurality of small, round openings 2.

Figure 6 depicts a dosage form 1 comprising a tablet-shaped core covered by a shell comprising a first shell portion 3a and a second shell portion 3b. First shell portion 3a comprises a plurality of openings 2a and 2b. Openings 2a are generally half-mooned shaped, while openings 2b are substantially smaller and circular.

Each opening may have dimensions, e.g., length, width, or diameter, in the range of about 0.1 % to about 100%, of the diameter of the dosage form, or of any dimension (e.g. diameter, length, or width) of a major face of the dosage form. The diameter or width of each opening is preferably from about 0.5% to about 5% of the diameter of the dosage form, or of any dimension (e.g. diameter, length, or width) of a major face of the dosage form. In certain embodiments the diameter or width of the openings may range from about 200 to about 2000 microns. The length of the openings may range from about 1% to about 100% of the diameter of the dosage form, or of the diameter of a major face of the dosage form.

In certain particular embodiments, the length or diameter of a major face of the dosage form is from about 10,000 to about 20,000 microns. In one particular embodiment, the length of the openings is from about 100 to about 20,000 microns. The depth of the openings is typically from about 75% to about 100% of the thickness of the shell at the location of the openings.

In certain embodiments, the thickness of the shell at the location of the openings typically ranges from about 20 to about 800 microns, e.g. from about 100 to about 400 microns. In one particular embodiment, the depth of the openings is from about 75 to about 400 microns. If a plurality of openings is present, they are typically spaced from one another by at least about one half, e.g. at least about one, times the smallest dimension of the smallest opening. The openings may have a variety of shapes, or be arranged in a variety of different patterns, and may have similar or different sizes, such as depicted in Figure 6.

In one embodiment, the size of the openings is small enough to prevent the core from being tasted, yet the number of openings is large enough to provide communication between a certain percentage of surface area of the core and the exterior of the dosage form.

In one particular embodiment, the plurality of openings is arranged with respect to one another such that the openings function as perforations, to separate a continuous portion, referred to herein as a "patch", of the shell from the dosage form at some time after contact of the dosage form with a suitable dissolution medium. The plurality of openings can be arranged in any pattern that meets the criterion that when each opening is connected to the next, a continuous line is formed, which encloses a portion of the shell to be separated from the remainder of the shell and dosage form during dissolution. For example, the openings may be arranged in the shape of a circle, oval, square, rectangle, triangle, pentagon, hexagon, heptagon, octagon, trapezoid, diamond, star, and the like. The plurality of openings forming such a pattern may be the same or different in shape and size, and the space between the openings may be similar to, or substantially larger than the width of each opening or the width of the smallest openings.

One embodiment is depicted in Figure 6 wherein the openings are arranged approximately in a circular pattern on the shell portion covering one face of the dosage form. In this example, the plurality of openings are of different shapes and sizes, with the larger openings serving to expedite the influx of water into the dosage form, and the smaller openings serving as additional weak points in the shell, e.g. "perforations", to facilitate the dissolution of the shell material in that area. When the dosage form of Figure 6 is placed in a dissolution medium, the circular "patch" of shell material surrounded by the openings separates from the dosage form, exposing the underlying core to the dissolution medium. This embodiment advantageously minimizes exposure of the core to the environment in the oral cavity (e.g. minimizing taste of the core by the patient), while simultaneously maximizing exposure of the core to the dissolution medium (e.g. gastro-intestinal fluids after ingestion).

The shell thickness at various locations may be measured using a microscope, for example, an environmental scanning electron microscope, model XL 30 ESEM LaB6, Philips Electronic Instruments Company, Mahwah, WI. The shell thickness is measured at 6 different locations on a single dosage form. The relative standard deviation (RSD) is calculated as the sample standard deviation, divided by the mean, times 100 as known in the art (i.e. the RSD is the standard deviation expressed as a percentage of the mean). The RSD in shell thickness provides an indication of the variation in the thickness of the shell on a single dosage form. In certain optional embodiments of the invention, the relative standard deviation in shell thickness is less than about 40%, e.g. less than about 30%, or less than about 20%.

The dosage forms of the invention provide immediate release of one or more active ingredients contained therein. The active ingredient or ingredients may be found within the core, the shell, or portions or combinations thereof. In one embodiment, at least one active ingredient is contained in the core.

In embodiments in which it is desired for the active ingredient to be absorbed into the systemic circulation of an animal, the active ingredient or ingredients are preferably capable of dissolution upon contact with a fluid such as water, gastric fluid, intestinal fluid or the like. In one embodiment, the dissolution characteristics of at least one active ingredient meet USP specifications for immediate release tablets containing the active ingredient. For example, for acetaminophen tablets, USP 24 specifies that in pH 5.8 phosphate buffer, using USP apparatus 2 (paddles) at 50 rpm, at least 80% of the acetaminophen contained in the dosage form is released therefrom within 30 minutes after dosing, and for ibuprofen tablets, USP 24 specifies that in pH 7.2 phosphate buffer, using USP apparatus 2 (paddles) at 50 rpm, at least 80% of the ibuprofen contained in the dosage form is released therefrom within 60 minutes after dosing. See USP 24, 2000 Version, 19 - 20 and 856 (1999). In another embodiment, at least about 70% the active ingredient can be detected in a suitable dissolution medium after 60 minutes of stirring at suitable conditions.

Accordingly, the shell, or a portion thereof, is readily soluble in gastrointestinal fluids. In a preferred embodiment in which the shell comprises a first and second shell portion, at least the first shell portion comprises openings therein, and is readily soluble in gastrointestinal fluids. In such embodiments, such shell or shell portion will preferably be breached or dissolved within 30 minutes in 900 ml water or 0.1 N HCl, or phosphate buffer solution at 37°C with stirring by a USP type 2 dissolution apparatus (Paddle method) at 50 or 100 rpm.

The shell or shell portion preferably comprises materials that exhibit rapid dissolution in gastro-intestinal fluids. For example, such shell or shell portion may comprise readily soluble materials selected from water-soluble or water swellable film formers, water-soluble or water swellable thickeners, crystallizable and non-crystallizable carbohydrates. In certain such embodiments, suitable water-soluble or water swellable film formers may be selected from water swellable cellulose derivatives, thermoplastic starches, polyalkylene glycols, polyalkylene oxides, and amorphous sugar glass, and combinations thereof. In certain other such embodiments, suitable film formers may be selected from film forming water soluble polymers such as for example water soluble vinyl polymers, water soluble polycarbohydrates, and water soluble copolymers; film-forming proteins, and combinations thereof. In certain other such embodiments, suitable thickeners may be selected from gelling polymers or hydrocolloids; gelling starches, and crystallizable carbohydrates. In certain other such embodiments, suitable non-crystallizable carbohydrates may be selected from polydextrose, starch hydrolysates, and non-crystallizable sugar alcohols. In one embodiment, the shell preferably comprises at least about 50%, preferably at least about 80%, most preferably at least about 90% of a material selected from film formers, gelling polymers, low-melting hydrophobic materials, non-crystallizable sugars or sugar alcohols, and mixtures thereof. In another embodiment, the shell comprises at least about 50%, preferably at least about 80%, most preferably at least about 90% of a material selected from film formers, gelling polymers, low-melting hydrophobic materials, and mixtures thereof.

In another particular embodiment, the shell comprises less than about 50%, preferably less than about 25%, most preferably less than about 5% of a crystallizable sugar.

In another embodiment, the dosage form is substantially free (i.e. less than 1% by weight, preferably less than about 0.1% by weight, based upon the shell weight) of charge control agents. As used herein, the term "charge control agents" refers to a material having a charge control function, such as those used for electrostatic deposition of coatings onto substrates. Such charge control agents include metal salicylates, for example zinc salicylate, magnesium salicylate and calcium salicylate; quaternary ammonium salts; benzalkonium chloride; benzethonium chloride; trimethyl tetradecyl ammonium bromide (cetrimide); and cyclodextrins and their adducts.

In certain optional embodiments, the shell itself or an outer coating thereon may contain active ingredient. In one particular embodiment, such active ingredient will be released immediately from the dosage form upon contact with suitable liquid media. In another embodiment, the shell comprises a first shell portion and a second shell portion. An outer coating resides upon the second shell portion, while the first shell portion comprises openings therein. Active ingredient may be released either immediately, or in a controlled, e.g. sustained, prolonged, extended manner, or in a delayed, e.g. pulsatile, or repeat action manner from the dosage form upon contact with suitable liquid media. In embodiments wherein the active ingredient is released immediately from the outer coating, the outer coating is also preferably readily soluble in gastrointestinal fluids, as described above.

The shell may be applied to the core by any suitable method, for example by spraying, dipping, enrobing, or molding. Suitable spray-coating methods are described in, for example, United States Patent Nos. 3,185,626, 4,683,256, 4,543,370, 4,643,894, 4,828,841, 4,725,441, 4,802,924, 5,630,871, and 6,274,162, the disclosures of which are all incorporated by reference herein. Suitable dipping methods are described in U.S. Patent Nos. 4,820,524, 5,538,125; 5,228,916; 5,436,026; 5,679,406, the disclosures of which are all incorporated by reference herein. Suitable enrobing methods are described in U.S. Patent Nos. 5,146,730 and 5,459,983. Suitable molding methods are described herein.

In certain optional embodiments of the invention, the core, the shell, or both are prepared by molding. In particular, the core, the shell, or both may be made by solvent-based molding or solvent-free molding. In such embodiments, the core or the shell is made from a flowable material optionally comprising active ingredient. The flowable material may be any edible material that is flowable at a temperature between about 37°C and 250°C, and that is solid, semi-solid, or can form a gel at a temperature between about -10°C and about 35°C. When it is in the fluid or flowable state, the flowable material may comprise a dissolved, dispersed, or molten component, and optionally a solvent such as for example water or organic solvents, or combinations thereof. The solvent may be partially or substantially removed by drying.

In one embodiment, solvent-based or solvent-free molding is performed via thermal setting molding using the method and apparatus described in copending U.S. patent application Serial No. 09/966,450, pages 57-63, or via zero cycle injection molding using the methods and apparatus described in copending U.S. Patent Application Serial No. 10/677,984, filed October 2, 2003, the disclosures of which areis incorporated herein by reference. In these embodiments, a core or shell is formed by injecting flowable form into a molding chamber. The flowable material preferably comprises a thermal setting material at a temperature above its melting point but below the decomposition temperature of any active ingredient contained therein. The starting material is cooled and solidifies in the molding chamber into a shaped form (i.e., having the shape of the mold).

According to these methods, the flowable material may comprise solid particles suspended in a molten matrix, for example a polymer matrix. The flowable material may be completely molten or in the form of a paste. The flowable material may comprise an active ingredient dissolved in a molten material. The flowable material may comprise solid particles dispersed in a fluid carrier. Alternatively, the flowable material may be made by dissolving a solid in a solvent, which solvent is then evaporated after the molding step.

In another embodiment, solvent-based or solvent-free molding is performed by thermal cycle molding using the method and apparatus described in copending U.S. patent application Serial No. 09/966,497, pages 27-51 or via zero cycle injection molding using the methods and apparatus described in copending U.S. Patent Application Serial No. 10/677,984, filed October 2, 2003, the disclosures of which areis incorporated herein by reference. Suitable molding is performed by injecting a flowable material into a molding chamber. The flowable material may comprise active ingredient and a thermoplastic material at a temperature above the set temperature of the thermoplastic material but below the decomposition temperature of active ingredient. The flowable material is cooled and solidifies in the molding chamber into a shaped form (i.e., having the shape of the mold).

In the thermal cycle molding method and apparatus of U.S. patent application Serial No. 09/966,497 a thermal cycle molding module having the general configuration shown in Figure 3 therein is employed. The thermal cycle molding module 200 comprises a rotor 202 around which a plurality of mold units 204 are disposed. The thermal cycle molding module includes a reservoir 206 (see Figure 4) for holding flowable material. In addition, the thermal cycle molding module is provided with a temperature control system for rapidly heating and cooling the mold units. Figures 55 and 56 depict the temperature control system 600.

The mold units may comprise center mold assemblies 212, upper mold assemblies 214, and lower mold assemblies 210, as shown in Figures 26-28, which mate to form mold cavities having a desired shape, for instance of a core or a shell surrounding one or more cores. As rotor 202 rotates, opposing center and upper mold assemblies or opposing center and lower mold assemblies close. Flowable material, which is heated to a flowable state in reservoir 206, is injected into the resulting mold cavities. The temperature of the flowable material is then decreased, hardening the flowable material. The mold assemblies open and eject the finished product.

In one optional embodiment of the invention, the shell is applied to the dosage form using a thermal cycle molding apparatus of the general type shown in Figures 28A-C of copending U.S. Application Serial No. 09/966,497 comprising rotatable center mold assemblies 212, lower mold assemblies 210 and upper mold assemblies 214. Cores are continuously fed to the mold assemblies. Shell flowable material, which is heated to a flowable state in reservoir 206, is injected into the mold cavities created by the closed mold assemblies holding the cores. The temperature of the shell flowable material is then decreased, hardening it around the cores. The mold assemblies open and eject the finished dosage forms. Shell coating is performed in two steps, each half of the dosage forms being coated separately as shown in the flow diagram of Figure 28B of copending U.S. Application Serial No. 09/966,939 via rotation of the center mold assembly.

In another optional embodiment of the invention, the shell is applied to the dosage form using a zero cycle molding apparatus of the general type shown in copending U.S. Application Serial No. 10/677,984 comprising rotatable center mold assemblies 212, lower mold assemblies 210 and upper mold assemblies 214. Cores are continuously fed to the mold assemblies. Shell flowable material, which is heated to a flowable state in reservoir 206, is injected into the mold cavities created by the closed mold assemblies holding the cores. The flowable material hardens around the cores. The mold assemblies open and eject the finished dosage forms. Shell coating is preferably performed in two steps, each half of the dosage forms being coated separately via rotation of the center mold assembly.

In one embodiment, the compression module of copending U.S. patent application Serial No. 09/966,509, pp. 16-27 may be employed to make the core and the shell is applied to the core using a thermal cycle molding module as described above. A transfer device as described in U.S. patent application Serial No. 09/966,414, pp. 51-57, the disclosure of which is incorporated herein by reference, may be used to transfer the cores from the compression module to the thermal cycle molding module. Such a transfer device may have the structure shown as 300 in Figure 3 of copending U.S. Application Serial No. 09/966,939. It comprises a plurality of transfer units 304 attached in cantilever fashion to a belt 312 as shown in Figures 68 and 69 of copending U.S. Application Serial No. 09/966,939. The transfer device rotates and operates in sync with the compression module and the thermal cycle molding module to which it is coupled. Transfer units 304 comprise retainers 330 for holding cores as they travel around the transfer device.

Suitable thermoplastic materials for use in or as the flowable material include both water-soluble and water insoluble polymers that are generally linear, not crosslinked, and not strongly hydrogen bonded to adjacent polymer chains. The thermoplastic material may be single materials, or may be mixtures of materials with solvent or plasticizer. Examples of suitable thermoplastic materials include those comprising water swellable cellulose derivatives, water insoluble cellulose derivatives, thermoplastic vinyl polymers, thermoplastic starches, thermoplastic polyalkylene glycols, thermoplastic polyalkylene oxides, and amorphous sugar-glass, and the like, and derivatives, copolymers, and combinations thereof. Examples of suitable thermoplastic water swellable cellulose derivatives include hydroxypropyl cellulose (HPC), hydroxypropylmethyl cellulose (HPMC), methyl cellulose (MC), and their combinations with water or other suitable solvents and/or plasticizers. Examples of suitable thermoplastic water insoluble cellulose derivatives include cellulose acetate (CA), ethyl cellulose (EC), cellulose acetate butyrate (CAB), cellulose propionate, and their combinations with suitable organic solvents and/or plasticizers. Examples of suitable thermoplastic vinyl polymers include polyvinyl alcohol (PVA) and polyvinyl pyrrolidone (PVP). Examples of suitable thermoplastic starches are disclosed for example in U.S. Patent No. 5,427,614. Examples of suitable thermoplastic polyalkylene glycols include polyethylene glycol. Examples of suitable thermoplastic polyalkylene oxides include polyethylene oxide having a molecular weight from about 100,000 to about 900,000 Daltons. Other suitable thermoplastic materials include sugar in the form on an amorphous glass such as that used to make hard candy forms.

It should be noted that, when used to make the shell, the flowable material must be a material readily soluble in gastrointestinal fluids, as described above.

In those embodiments in which the shell is prepared using a solvent-free molding process, the shell typically comprises at least about 30 percent, e.g. at least about 45 percent by weight of a thermal-reversible carrier. The shell may optionally further comprise up to about 30 weight percent total of various plasticizers, adjuvants and excipients.

In those embodiments in which the shell is prepared using a solvent-based molding process, the shell typically comprises at least about 10 weight percent, e.g. at least about 12 weight percent or at least about 15 weight percent or at least about 20 weight percent or at least about 25 weight percent of a film-former. The shell may again also optionally further comprise up to about 30 weight percent total of various plasticizers, adjuvants, and excipients.

The total weight of the shell is preferably about 20 percent to about 400 percent of the total weight of the cores. In embodiments wherein the shell is prepared by a solvent-free molding process, the total weight of the shell is typically from about 50 percent to about 400 percent, e.g. from about 75 percent to about 400 percent, or about 100 percent to about 200 percent of the total weight of the cores. In embodiments wherein the shell is prepared by a solvent-based molding process, the total weight of the shell is typically from about 20 percent to about 100 percent of the total weight of the cores.

In embodiments in which the shell is applied to the core by molding, at least a portion of the shell surrounds the core such that the shell inner surface resides substantially conformally upon the core outer surface. As used herein, the term "substantially conformally" shall mean that the inner surface of the shell has peaks and valleys or indentations and protrusions corresponding substantially inversely to the peaks and valleys of the outer surface of the core. In certain such embodiments, the indentations and protrusions typically have a length, width, height or depth in one dimension of greater than 10 microns, say greater than 20 microns, and less than about 30,000 microns, preferably less than about 2000 microns.

In those embodiments in which solvent-free molding is employed, the flowable material may comprise a thermal-reversible carrier. Suitable thermal-reversible carriers for use in making a core, the shell or both by molding are thermoplastic materials typically having a melting point below about 110°C, more preferably between about 20 and about 100°C.

Examples of suitable thermal-reversible carriers for solvent-free molding include thermoplastic polyalkylene glycols, thermoplastic polyalkylene oxides, low melting hydrophobic materials, thermoplastic polymers, thermoplastic starches, and the like. Preferred thermal-reversible carriers include polyethylene glycol and polyethylene oxide. Suitable thermoplastic polyalkylene glycols for use as thermal-reversible carriers include polyethylene glycol having molecular weight from about 100 to about 20,000, e.g. from about 100 to about 8,000 Daltons. Suitable thermoplastic polyalkylene oxides include polyethylene oxide having a molecular weight from about 100,000 to about 900,000 Daltons.

Suitable low-melting hydrophobic materials for use as thermal-reversible carriers include fats, fatty acid esters, phospholipids, and waxes which are solid at room temperature, fat-containing mixtures such as chocolate; and the like. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono-, di-, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides.

Examples of suitable phospholipids include phosphotidyl choline, phosphotidyl serene, phosphotidyl enositol, and phosphotidic acid. Examples of suitable waxes that are solid at room temperature include camauba wax, spermaceti wax, beeswax, candelilla wax, shellac wax, microcrystalline wax, and paraffin wax. \

Suitable thermoplastic polymers for use as thermal-reversible carriers include thermoplastic water swellable cellulose derivatives, thermoplastic water insoluble polymers, thermoplastic vinyl polymers, thermoplastic starches, and thermoplastic resins, and combinations thereof.

Suitable thermoplastic water swellable cellulose derivatives include hydroxypropylmethyl cellulose (HPMC), methyl cellulose (MC), carboxymethylcellulose (CMC), cross-linked hydroxypropylcellulose, hydroxypropyl cellulose (HPC), hydroxybutylcellulose (HBC), hydroxyethylcellulose (HEC), hydroxypropylethylcellulose, hydroxypropylbutylcellulose, hydroxypropylethylcellulose, and salts, derivatives, copolymers, and combinations thereof.

Suitable thermoplastic water insoluble polymers include ethylcellulose, polyvinyl alcohols, polyvinyl acetate, polycaprolactones, cellulose acetate and its derivatives, acrylates, methacrylates, acrylic acid copolymers, and the like and derivatives, copolymers, and combinations thereof.

Suitable thermoplastic vinyl polymers include polyvinylacetate, polyvinyl alcohol, and polyvinyl pyrrolidone (PVP). Examples of suitable thermoplastic starches for use as thermal-reversible carriers are disclosed for example in U.S. Patent No. 5,427,614. Examples of suitable thermoplastic resins for use as thermal-reversible carriers include dammars, mastic, rosin, shellac, sandarac, and glycerol ester of rosin. In one embodiment, the thermal-reversible carrier for making a core by molding is selected from polyalkylene glycols, polyalkylene oxides, and combinations thereof.

In embodiments in which the shell comprises an active ingredient intended to have immediate release from the dosage form, the shell is preferably prepared via solvent-free molding. In such embodiments a thermal-reversible carrier is employed in the flowable material to make the shell, said thermal-reversible carrier preferably selected from polyethylene glycol with weight average molecular weight from about 1450 to about 20000, polyethylene oxide with weight average molecular weight from about 100,000 to about 900,000, and the like.

In one embodiment of the invention, the shell is applied to the core by enrobing. In enrobing methods, cast films are applied to opposite sides of a core, i.e., a compressed tablet, using rotating dies, and are sealed together in an essentially edge-to-edge manner at a seal line that extends around the core at a desired place. The dies are positioned such that the surfaces are in abutting relationship with one another, thereby forming a nip in between. Each of the dies has a series of matching recesses on its circumferential surface. As the dies rotate, the films are joined and fused together at the nip between the dies where a pair of matching recesses forms a pocket into which a core is dropped by a metered feed mechanism. As the dies continue to rotate, the core urges the films into the interior of the recesses in the dies, and the core is thereby securely enveloped and enrobed by the films, while the films continue to be joined about the core by the dies. Simultaneously with the fusing of the films about the core, the enrobed core is pinch cut from the films by the rotary dies, whereupon it separates from the films in the form of an individual enrobed dosage form.

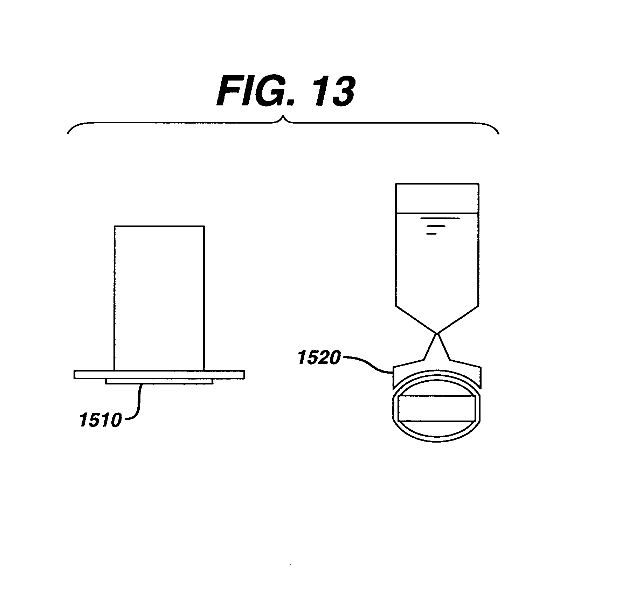

Useful enrobing methods and apparatuses are described for example in U.S. Patent Nos. 5,146,730 and 5,459,983, the disclosures of which are incorporated herein by reference. Referring to the '730 patent and Figure 13 in particular, cast films 36 and 37 are formed on rotating casting drums 42 and 43, which are cooled by a coolant. The films are fed through a series of rotating drums to drum-like dies 38 and 39, which rotate synchronously with one another. Dies 38 and 39 are symmetrically disposed relative to one another about a functional center plane 54 of the overall enrobing apparatus. Films 36 and 37 contact each other at the nip between dies 38 and 39. Here, the cores are enrobed with coating, and the two films are sealed together and cut.

Specifically, a web is created as films 36 and 37 converge and are squeezed together. The films self-adhere together. After emerging from between dies 38 and 39, the web containing the enrobed cores passes between a pair of mangle rolls 63. The web is stretched, and the enrobed cores self separate and drop into product receptacles 65.

Referring to Figure 20 of the '730 patent, each die 38, 39 has a plurality of recesses 108, which cooperate with corresponding recesses in the other die. The cavities of these recesses are shaped to receive a single core. The cavities are defined by ribs 109 that close together to cut the enrobed cores from the web. Teeth 115 on the edges of the dies 38, 39 grip the films 36, 37.

The enrobed cores can be washed and dried, and optionally further processed as desired.

In another embodiment, the shell is again applied to the core by enrobing, however the films used to make the shell each comprise visually distinct portions, for example stripes. The films are applied by the enrobing method described in commonly assigned, copending U.S. Application Serial Nos. 10/146471, filed May 15, 2002 and 10/146722, filed May 15, 2002. Referring generally to Figures 9-11 therein, the film casting apparatus 30 for this method of enrobing comprises a casting drum 34, the exterior surface 36 of which is cooled to at least partially solidify coating materials coming into contact therewith. A multi-chamber slit extruder 38 deposits film onto the casting drum 34. The extruder 38 comprises partitions 58, 60, 62 that divide the interior 44 into four chambers 64, 66, 68, 70. The chambers 64, 66, 68, 70 can each contain visually different coating materials, i.e., different colored coating materials. Each partition has a tapered blade edge 94, 96, 98 to control the flow of coating material onto the casting drum 34. The coating materials are supplied to the chambers from for example feeder pipes 72, 74. An open slit 78 is located at the bottom of the slit extruder 38. Slit 78 communicates with each chamber 64, 66, 68, 70. The slit extruder is heated to heat the coating materials to a flowable state, which depending on the material may be in the range of about 40 to 250° C.

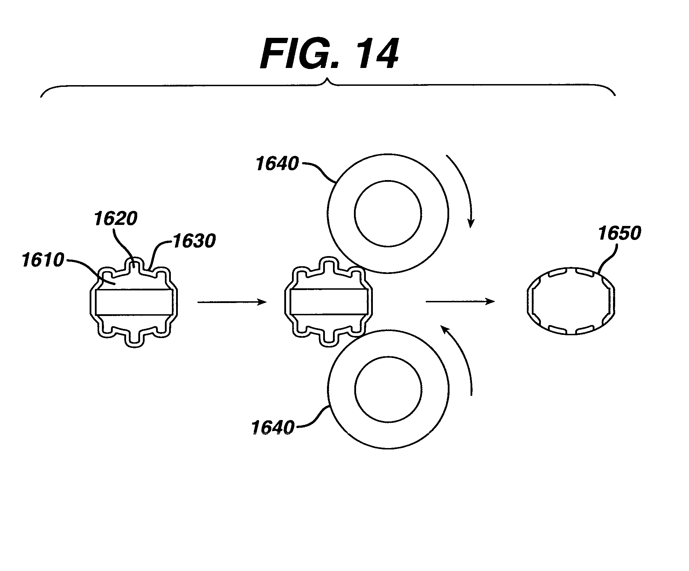

Referring to Figures 14-16 of the 10/146471, filed May 15, 2002 application, an enrobing apparatus 102 for use with cast films having visually distinct portions similar to that described in U.S. Patent Nos. 5,146,730 and 5,459,983 may be used. In particular, cast films 32, 32' are moved in a continuous manner, by a series of rollers 106, 108, 110, 106', 108', 110' toward a pair of coacting rotary dies 112, 112', which are positioned symmetrically on either side of the central plane of symmetry 104 of the apparatus 102. The rotary dies 112, 112' rotate on their axes of rotation AR, AR', respectively, thereby forming a nip in between. The nip between the rotary dies 112, 112' lies in the aforesaid central plane of symmetry 104 and the striped films 32, 32' are passed therethrough.

The enrobing apparatus 102 also includes a core dispensing means 118, which holds a supply of cores 10 and dispenses them to the nip in a timed manner. The core dispensing means 118 is also aligned with the central plane of symmetry 104. The core dispensing means 118 orients and dispenses each core 10 such that the core 10 simultaneously contacts the contact surfaces 32a, 32a' of the converging striped films 32, 32' as the core 10 enters the nip, with its transverse plane of symmetry 16 lying in the central plane of symmetry 104 of the enrobing apparatus 102, and the color transitions 92a, 92a' of the films 32, 32', respectively, lying in the conjugate plane of symmetry 18 of the core 10. The films 32, 32' are then stretched around the opposite sides of each core 10 symmetrically.

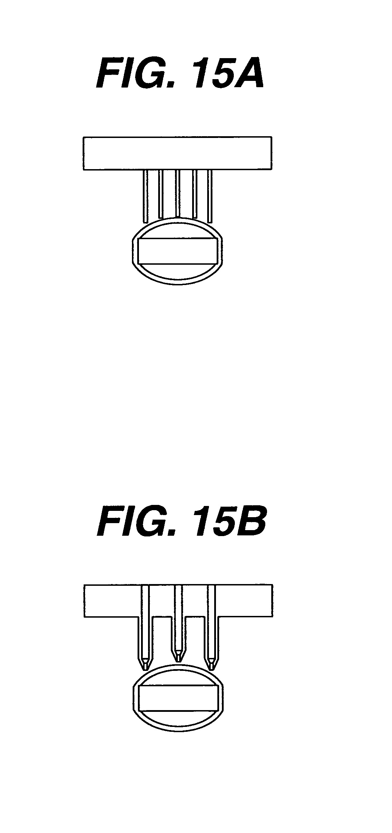

Figure 15 of the 10/146471, filed May 15, 2002 application shows the proper positioning of the cores 10 in between the striped films 32, 32' as they enter the nip between the dies 112, 112' and relative to the color transitions 92a, 92b, 92c, 92a', 92b', 92c' of each film 32, 32'. All of the color transitions 92a, 92b, 92c, 92a', 92b', 92c' are aligned with the conjugate plane of symmetry 16 of a corresponding core 10.

Furthermore, the enrobing apparatus 102 preferably includes registering means 120 (shown schematically in Figure 14 of 10/146471, filed May 15, 2002 application) for ensuring that the colored stripes (not shown) of the films 32, 32' are properly aligned with one another prior to passage between the rotary dies 112, 112'. The registering means 120 also ensures that the positions of the dispensed cores 10 are appropriate relative to the color transitions 92a, 92b, 92c, 92a', 92b', 92c', such that the transition between the colors on the resulting products 122 are properly matched with one another and the conjugate plane of symmetry 18 of each core 10.

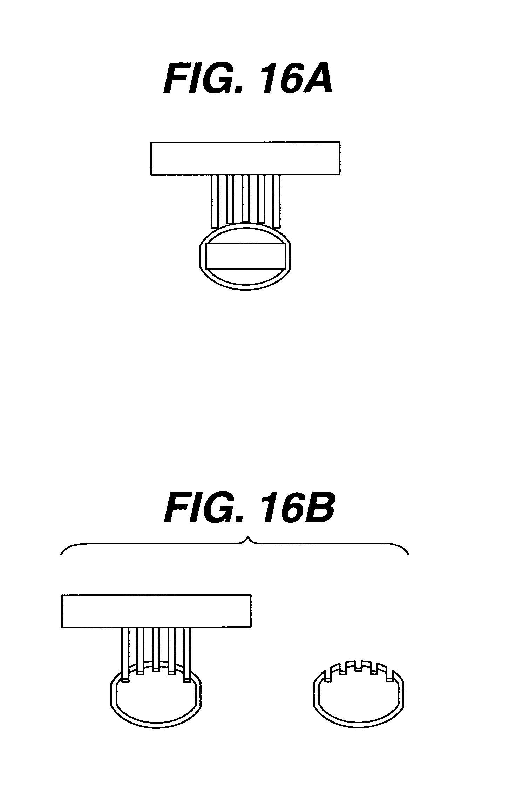

An enlarged schematic perspective view of the drum-like rotary dies 112, 112' is provided in Figure 16 of the 10/146471, filed May 15, 2002 application. The rotary dies 112, 112' are substantially identical to one another, each having an exterior circumferential surface 124, 124' with a series of recesses 126, 126' thereon. Each recess 126, 126' is arranged such that its length 130, 130' is aligned parallel to the axis of rotation AR, AR' of its respective rotary die 112, 112'. Each recess 126 on one die 112 cooperates with a corresponding recess 126' on the other die 112'. In addition, the number of rows of recesses 126, 126' should correspond to the number of color transitions 92a, 92b, 92c, 92a', 92b', 92c' between the stripes 84, 86, 88, 90, 84', 86', 88', 90' on the striped films 32, 32', respectively, that pass between the dies 112, 112'.

The orientation of the striped films 32, 32' as they pass between the rotary dies 112, 112' is such that, for example, red stripes 84, 88 of one film 32 are matched with the red stripes 84', 88' of the other film 32' and yellow stripes 86, 90, 86', 90' of each film 32, 32', respectively, are similarly matched with one another. The registering means 120 of the enrobing apparatus 102 may be used to facilitate the orientation of the films 32, 32' such that the matching and alignment of the color transitions of each film are improved.

As the rotary dies 112, 112' rotate, the cores 10 are dispensed to the nip between the dies 112, 112' such that they are oriented with their lengths aligned parallel to the axes of rotation AR, AR' of the dies 112, 112' and each core 10 is thereby properly aligned to be received between a pair of coacting recesses 126, 126'. The rotary dies 112, 112' continue to rotate and the films 32, 32' are sealed to each other by the raised rims 128, 128' of the coacting recesses 126, 126', around the core 10 thereby forming a film seam 134, which lies in the transverse plane of symmetry 16 of the core 10. The raised rims 128, 128' also cut through the bonded films 32, 32', at the film seam 134 around each enrobed core 10, thereby releasing the enrobed core products 122, from the bonded films 32, 32'.

The film seam 134 created may comprise abutting film edges. It is also possible to have a film seam 134 wherein the cut edge of one film 32 slightly overlaps the cut edge of the other film 32' by an amount approximately equal to the thickness of the films 32, 32'. Alternatively, the film seam 134 could be formed such that the cut edges of the films 32, 32' are aligned with one another about the core 10, but are spaced apart slightly by a distance that is approximately equal to the thickness of the films 32, 32'.

If aesthetically desired, the films 32, 32' may be aligned such that the resulting products 122 have a film seam 134 wherein a stripe of one color (for example, a red stripe 84) (or visual distinction) of one film 32 abuts or overlaps a stripe of another color (for example, a yellow stripe 90') (or visual distinction) of the other film 32' to form a product 122 having a "checkerboard pattern", i.e., having four quadrants of alternating red and yellow colors (or other visual distinctions).

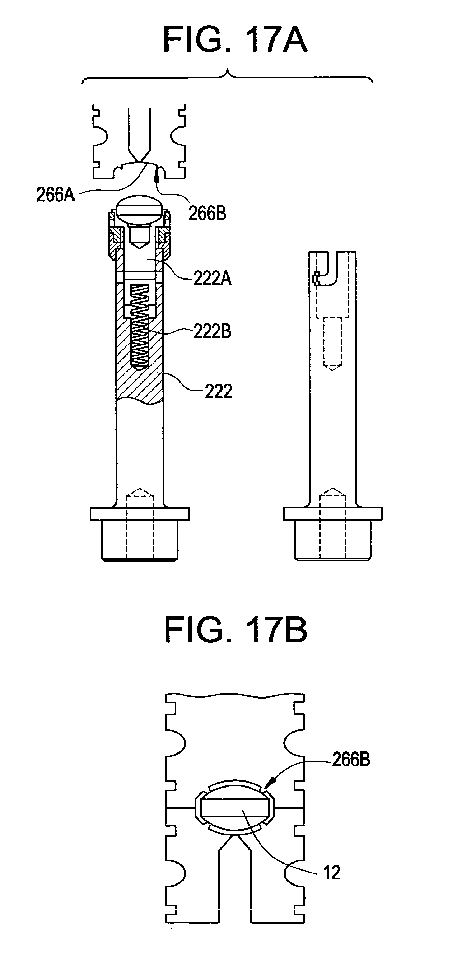

Figures 17-19 of the 10/146471, filed May 15, 2002 application depict an alternative film casting apparatus 136 for use with enrobing processes. The alternative film casting apparatus 136 includes film receiving means, such as a conventional metal casting belt 140 that is mounted onto two rotating drums 142, 144, for receiving the film 138 being cast thereon. The rotating drums 142, 144 rotate, thereby causing the casting belt 140 to move in the directions indicated by the arrows J and K. A warming plate 148 may be positioned adjacent to the casting belt 140 to warm the casting belt 140 prior to casting film thereon. A cooling plate 150 is positioned adjacent to the casting belt 140 to cool the casting belt 140 after film is cast thereon.

The alternative film casting apparatus 136 further includes film depositing means, such as a reciprocating multi-chamber slit extruder 146, for depositing the film 138, in a semi-continuous manner, onto the casting belt 138. As with the slit extruder 38 discussed above, the reciprocating slit extruder 146 includes interior partitions 152, 154, 156 that form interior chambers 158, 160, 162, 164 for holding visually distinct coating material 166, 168 therein. A slit 170 is also provided, through which the coating materials 166, 168 flow out of the chambers 158, 160, 162, 164 and onto the casting belt 140, thereby creating a striped film 138.

The reciprocating slit extruder 146 also includes supply means, such as feeder pipes 182, 184 for supplying the coating materials 166, 168 to each of the chambers 158, 160, 162, 164 and flow control means, such as a slidable gate 180 for controlling the flow of the coating materials 166, 168 from the chambers 158, 160, 162, 164. Each of the interior partitions 152, 154, 156 of the reciprocating slit extruder 146 has stripe control means, such as a tapered blade edge, to control the flow of the coating materials 166, 168 exiting the chambers 158, 160, 162, 164. One notable difference between the slit extruder 38 described above and the present reciprocating slit extruder 146 is that reciprocating slit extruder 146 is connected to a conventional motor such that it moves reciprocatingly in the directions indicated by arrow M in Figure 18 of the 10/146471, filed May 15, 2002 application.

Initially, feeder pipes 182, 184 supply coating materials 166, 168 of two colors, such as red and yellow, respectively, to alternate chambers 158, 160, 162, 164, respectively, of the reciprocating slit extruder 146. Within chambers 158, 160, 162, 164 the coating materials 166. 168 become or are maintained as liquid and flowable. Cooling plate 150 at least partially solidifies the coating material 166, 168 upon physical contact with the surface of the casting belt 140 to form the transversely striped film 138.

After the coating materials 166, 168, the warming plate 148 and the cooling plate 150 have attained their desired temperatures, a portion 198 of the casting belt 140 is warmed by the warming plate 148 and is then advanced by the rotating drums 142, 144 to a position underneath the reciprocating slit extruder 146. The slidable gate 180 is then moved to a position, which opens the slit 170 to the thickness that is desired for the striped film 138. While the rotating drums 142, 144 and the casting belt 140 remain stationary, the coating materials 166, 168 flow out of the chambers 158, 160, 162, 164, along the tapered blade edges (not shown), through the slit 170 and onto the warmed portion 198 of the casting belt 140, which briefly maintains the coating material 166, 168 in a substantially liquid, flowable state.

Simultaneously with the flow of the coating materials 166, 168 onto the casting belt 140, the reciprocating slit extruder 146 is moved from a first position 194 to a second position 196, where it is temporarily halted. As soon as the reciprocating slit extruder 146 reaches its second position 196, the slidable gate 180 is moved to a closed position, thereby blocking the slit 170 and temporarily halting the flow of coating materials 166, 168, which results in the formation of a film segment 200. The film segment 200 has alternating, transversely oriented red stripes 172, 176 and yellow stripes 174, 178 with straight and consistent color transitions 186, 188, 190 therebetween.

Next, the rotating drums 142, 144 move the casting belt 140, such that the film segment 200 is moved, and a newly warmed portion of the casting belt 140 is positioned beneath the reciprocating slit extruder 140. The first film segment 200 is cooled while the second film segment is cast onto the casting belt 140.

When it is desired to cast a subsequent film segment, with the reciprocating slit extruder 146, now in its second position 196, and the casting belt 140 held stationary, the slidable gate 180 is again moved to a position which opens the slit 170 by an amount that is equal to the thickness desired for the striped film 138. As coating materials 166, 168 flow out onto the casting belt 140, the reciprocating slit extruder 146 is moved from its second position 196 back to its first position 194, where it is again temporarily halted. As the coating materials 166, 168 are being cast onto the casting belt 140, the first edge of the new film segment will meet and bond with the second edge 204 of the first film segment 200. After the reciprocating slit extruder 146 returns to its first position 194, the slidable gate 180 is again moved to its closed position, thereby blocking the slit 170 and temporarily halting the flow of coating materials 166, 168, which results in the creation of a new film segment that is bonded to the first film segment 200.

The foregoing process steps are repeated continuously, resulting in a film casting process that is semi-continuous and which produces a continuous ribbon of transversely striped film 138. The transversely striped film 138 is continuously removed from the casting belt 140 by a scraper or other device and then fed into the rotary die enrobing apparatus 102 for enrobing cores 10 as described above. However, because the alternative film casting apparatus 136 produces film 138 having stripes that are transversely oriented, rotary dies 208, 208' have recesses 210, 210' which are oriented such that their lengths are aligned perpendicularly to the axes of rotation of their respective rotary dies. In addition, the core dispensing means used herewith orients and dispenses each core 10 to the nip between the dies 208, 208' end-first, i.e., such that one of the ends 12, 14 of each caplet 10 simultaneously contacts the converging films 138, 138' as the core 10 enters the nip.

Figures 22-30 of the 10/146471, filed May 15, 2002 application are directed to an alternative enrobing apparatus for making films with visually distinct portions, which includes the alternative film casting apparatus 136 described above. A transversely striped film is fed, along with cores, into the alternative enrobing apparatus to produce bi-colored products, each having a film seam that only partially circumscribes the core and which lies in a reference plane that is different from the reference plane in which the color transition of the product lies.

The alternative enrobing apparatus includes a conveyor system 220 that comprises a series of horizontally-oriented rollers 222 and pairs of rollers, 224, 226, 228 for supporting and conveying the transversely striped film 138. A core dispensing means 230 is positioned above the conveyor system 220 and the film 138 for the purpose of dispensing cores 10 onto the film 138 in the required orientation with respect to the color transitions 186, 188, 190. The core dispensing means 230 includes slat feeders 234, 236 that orient the cores as required for proper positioning onto the film 138. The core dispensing means 230 further includes a core positioning slat 238 and a core plunger 240 positioned above the positioning slat 238.

Cores 10 are fed from hopper 232, through the slat feeders 234, 236, to the positioning slat 238. The cores 10', 10", 10''' are lined up, end 12 to end 14 and, thereby, each core is moved along the positioning slat 238 in a substantially continuous manner by the core behind it. When a core 10' is pushed beyond the support rails 248, 250 and is no longer supported thereby, and when the position of a color transition 186 of the film 138 lies in the conjugate plane of symmetry 18' of core 10', a registering means 242 signals a motor, which moves the plunger 240 up and down until the core 10' contacts and rests upon the film 138. When plunger 240 reaches its uppermost position, it momentarily stops until the next core 10" is moved beyond support rails 248, 250 contained within the positioning slat 238. The foregoing events are repeated continuously as long as cores 10 are fed and moved through the positioning slat 238.

The beginning portion of the conveyor system 220, i.e., the portion that is located between the alternative film casting apparatus 136 and a short distance on the opposite side of the core positioning slat 238, is comprised of horizontally-oriented rollers 222. Film 138 is moved by the horizontally-oriented rollers 222 along this beginning portion. The remaining portion of the conveyor system 220, which is located between a short distance past the positioning slat 238 and the rotary dies 260, 262, is comprised of pairs of rollers 224, 226, 228. As shown schematically in Figure 26 of the 10/146471, filed May 15, 2002 application, the individual rollers of sequential pairs of rollers 224, 226, 228 are gradually and sequentially pivoted upward from the horizontal plane, in increments of about 10 degrees for each successive pair of rollers 224, 226, 228, starting proximate to the positioning slat 238. Accordingly, as the film 138 approaches the rotary dies 260, 262, the film 138 is folded longitudinally about the cores 10.

The rotary dies 260, 262 rotate and cooperate with one another to form a nip therebetween, into which the cores 10 and the film 138 are fed. Likewise, each of the dies 260, 262 have recesses 282, 284, arranged circumferentially in a row on the surface of each die 260, 262. The recesses 282, 284 each have raised rims for sealing and cutting the bonded film 138 about the cores 10, thereby enrobing the cores 10. The rotary dies 260, 262 however, are oriented such that they rotate in the horizontal plane, rather than in the vertical plane as do the previously discussed rotary dies 112, 112', 208, 208'. Furthermore, when the cores 10 are fed into the nip, the film 138 is folded and partially bonded about them. Furthermore, the partially enrobed cores 10 are fed successively, i.e., one-by-one, into the nip between the dies 260, 262.

The shell may also be applied to the core using a vacuum forming apparatus. Commonly assigned, copending U.S. Patent Application Serial Nos. 10/146471, filed May 15, 2002 and 10/146722, filed May 15, 2002 disclose such apparatuses.

With reference to Figures 31-44 of the 10/146471, filed May 15, 2002 application, a vacuum forming apparatus 292 includes a first plurality of individual porous platens 294, a first conveyor system 296 and a second conveyor system 298. The first and second conveyor systems 296, 298 are arranged in series with one another, thereby creating a single path along which the porous platens 294 are moved in semi-continuous fashion. The first and second conveyer systems 296, 298 are provided with conventional vacuum sources that apply a vacuum to each of the porous platens 294 while they are moved along the apparatus.

The vacuum forming apparatus 292 further includes a second plurality of individual porous platens 304 and a third conveyor system 306 that moves porous platens 304 along a second path, which is shown by the arrow GG in Figure 31. The third conveyor system 306 is positioned between the first and second conveyor systems 296, 298.

A rotating mechanism 308 is also positioned between the first and second conveyor systems 296, 298. The rotating mechanism 308 simultaneously holds together two of platens, i.e., one platen 294 and a corresponding platen 304, and rotates them together, such that the platen 304, first on top, is inverted and positioned on the bottom.

Each porous platen 294, 304 has at least one recess 310, 312, respectively, sized and shaped to temporarily but snugly receive therein a core 10 to be enrobed.

It is possible to achieve multi-colored enrobed products having color transitions oriented in a variety of different ways using different combinations of the platens with transversely and longitudinally striped films. However, within a particular vacuum forming apparatus, the orientation of the recesses 310, 312 in all of the platens 294, 304 must be longitudinal, or, alternatively, the orientation of the recesses 310, 312 in all of the platens 294, 304 must be transverse (or otherwise aligned with the orientation of the stripes on the films).

The vacuum forming apparatus 292 further includes a first pair of rollers 338, 340 having a film 342 mounted thereon. The first pair of rollers 338, 340 is positioned proximate to the first conveyor system 296 such that the first film 342 is suspended above the first plurality of porous platens 294. A second pair of rollers 344, 346 having a second film 348 mounted thereon is positioned proximate to the second conveyor system 298 such that the second film 348 is also suspended above the first plurality of porous platens 294

The vacuum forming apparatus 292 also includes a first registering device 350 positioned proximate to the first conveyor system 296 for properly positioning the first film 342 relative to a core 10 that is positioned within the recess 310 of the porous platen 294. A second registering device 352 is positioned proximate to the second conveyor system 298 for properly positioning the second film 348 relative to a partially enrobed core 10 that is positioned within the recess 310 of another of the porous platens 294.

The vacuum forming apparatus 292 also includes a first ring press 354 and a first film cutter 356 that are positioned proximate to the first conveyor system 296 and move in a reciprocating fashion. Additionally, a second ring press 358 and a second film cutter 360 are positioned proximate to the second conveyor system 298. The first and second ring presses 354, 358 each have an open configuration, such as an O-shape or an oval shape, as viewed from above, such that there is formed a passageway 362, 364, respectively, therethrough. Each of the ring presses 354, 358 also has a contacting edge 366, 368, respectively, configured to contact the first and second films 342, 348, respectively, without damaging them. Each of the ring presses 354, 358 is sized and shaped such that the contacting edges 366, 368 circumscribe a core 10 therein.

The first and second film cutters 356, 360 each have a recess 370, 372, respectively, that is sized and shaped to receive therein a portion of a partially enrobed core 10 which already has a film coating applied thereto. The first film cutter 356 is oriented to face the porous platen 294 positioned thereunder, while the second film cutter 360 is oriented to face the porous platen 304 positioned thereunder. The first and second film cutters 356, 360 also move reciprocatingly.

The first film 342 is mounted onto a first pair of rollers 338, 340 and stretched therebetween, such that the first film 342 is positioned between the first conveyor system 296 and the first plurality of porous platens 294 on one side, and the first ring press 354 and the first film cutter 356 on the other side. Similarly, the second film 348 is mounted onto a second pair of rollers 344, 346 and stretched therebetween, such that the second film 348 is positioned between the second conveyor system 298 and the first plurality porous platens 294 on one side, and the second ring press 360 and the second film cutter 362 on the other side.

Initially, the first, second and third conveyor systems 296, 298, 306 are set into motion, thereby moving the porous platens 294, 304 in the directions indicated by the arrows EE, FF, GG, respectively, in Figure 31 of the 10/146471, filed May 15, 2002 application. The first conveyor system 296 moves one of the porous platens 294 to a position that is immediately prior to the first station 378. A core 10 is placed into the recess 310 of this porous platen 294 and held firmly in the recess 310 by the aforementioned vacuum, which is continuously applied to the platen 294 and all others on the conveyor 298, by a first vacuum source 300.

Platen 294 is next moved by the first conveyor system 296 to the first station 378. Movement of the platen 294 ceases temporarily when the first registering device 350 confirms that the core 10 is properly positioned. While the platen 294 is momentarily stationary, hot air is blown through the first ring press 354, thereby softening the first film 342 to a formable state. The first ring press 354 is then moved, such that the contacting edge 366 of the first ring press 354 presses the first film 342 onto the working surface 314 of the platen 294 and into contact with the top half of the core 10.

The heated first film 342 is simultaneously pulled onto the core 10 and thereby made to conform to the shape of the top half of the core 10 by the aforementioned vacuum. Thereafter, the first ring press 354 is retracted and platen 294 is moved to the second station 380, where it is temporarily stopped. While the platen 294 and the core 10 are temporarily stationary, cold air is blown onto the first film 342 and core 10, thereby cooling and molding the first film 342 into conformity with the top half of the core 10.

Referring to Figures 36-44 of the 10/146471, filed May 15, 2002 application, after sufficient time has passed to cool and mold the first film 342 onto the core 10, the platen 294 and partially enrobed core 10 are moved a predetermined distance by the first conveyor system 296 to the third station 382 of the vacuum forming apparatus 292 and temporarily halted there such that the partially enrobed core 10 is aligned with the recess 370 and the cutting edge 374 of the first film cutter 356. The first film cutter 356 is moved until the partially enrobed core 10 is received snugly within the recess 370 and the tapered cutting edge 374 contacts and cuts through the first film 342 closely around the perimeter of the partially enrobed core 10. The first film cutter 356 is then moved away from the platen 294.

The platen 294 and partially enrobed core 10 are next moved to the fourth, or rotating, station 384 of the vacuum forming apparatus 292 and, again, temporarily stopped, whereupon the partially enrobed core 10 is transferred to one of the platens 304, as follows. The third conveyor system 306 moves one of the platens 304 into position at the rotating station 384, such that it is inverted relative to the platen 294 carrying the partially enrobed core 10 thereon. After the platen 294 is moved a predetermined distance, such that the partially enrobed core 10 is aligned with the recess 312 of the inverted platen 304, the first conveyor system 296 holds the platen 294 and partially enrobed core 10 temporarily stationary at the rotating station 384. The platen 304 is then moved toward the partially enrobed core 10 until the partially enrobed core 10 is held within the recesses 310, 312 of both of the platens 294, 304 (as shown in Figures 31 and 40 of the 10/146471, filed May 15, 2002 application). The vacuum being applied to the porous platen 294 is discontinued and the platens 294, 304 are rotated with the partially enrobed core 10 therebetween, whereupon the platen 294 holding the core 10 is inverted, and the platen 304 is moved into a right-side-up position. The now inverted platen 294 is now moved away from the right-side-up platen 304 and becomes one of the platens 304 moving along the path shown by the arrow GG in Figure 31 of 10/146471, filed May 15, 2002 application. The right-side-up platen 304 is next moved onto the second conveyor system 298 and becomes one of the platens 294 moving along the path shown by the arrows EE, FF in Figure 31 of the 10/146471, filed May 15, 2002 application. Next, a vacuum is applied to partially enrobed core 10, thereby holding the partially enrobed core 10 within the recess 310 of the platen 294, which is now moving on the second conveyor system 298, such that the uncovered portion of the partially enrobed core 10 is exposed.

Platen 294 and partially enrobed core 10 are moved by the second conveyor system 298 to the fifth station 386 of the vacuum forming apparatus 292. Movement of the platen 294 ceases temporarily when the second registering device 352 confirms that the partially enrobed core 10 is properly positioned relative to the second film 248. While the platen 294 is momentarily stationary, hot air is blown through the second ring press 358 to soften the second film 348 to a formable state. The second ring press 358 is then moved such that the contacting edge 368 of the second ring presses 358 contacts and presses the second film 348 onto the working surface 314 of the platen 294 and into contact with the uncovered portion of the partially enrobed core 10.

The heated second film 248 is then pulled onto the core 10 by a vacuum applied by the second vacuum source 302, thereby conforming the second film 248 to the shape of the uncovered portion of the core 10. Thereafter, the second ring press 358 is retracted and the platen 294, having the enrobed core 10 held in its recess 310 by the vacuum, is now moved to the sixth station 388 and temporarily stopped there. It is noted that, as shown in Figure 42 of the 10/146471, filed May 15, 2002 application, the second film 348 partially overlaps the cut edge of the first film 342 that has already been applied to the core 10. While the platen 294 and the core 10 are temporarily stationary, cold air is now blown onto the second film 248 and core 10, thereby cooling and molding the second film 348 into conformity with the core 10.

After sufficient time has passed to cool and mold the second film 348 onto the core 10, the platen 294 and the enrobed core 10 are moved a predetermined distance by the second conveyor system 298 to the seventh station 390 of the vacuum forming apparatus 292 and temporarily halted there such that the enrobed core 10 is aligned with the recess 372 and the cutting edge 376 of the second film cutter 360. The second film cutter 360 is moved until the enrobed core 10 is received snugly within the recess 372 and the cutting edge 376 contacts and cuts through the second film 248 closely around the perimeter of the enrobed core 10. The second film cutter 360 is then moved away from the platen 294.

The platen 294 and fully enrobed core 10 are moved by the second conveyer system 298 away from the seventh station 390. After the platen 294 and enrobed core 10 are past the seventh station 390, the vacuum being applied to the platen 294 is ceased, thereby releasing the enrobed product 404 from the recess 310.

Alternatively, platens having recesses that are each circumscribed by a raised cutting ridge capable of cleanly cutting the first and second films 342, 348 may be used. Alternatively, instead of first placing the caplet 10 into the recess 310 of the first platen 294, the first film 342 could be laid across a first platen and then warm air could be blown onto the first film 342 to soften it to a formable state. Then, a vacuum could be applied through the platen to pull the first film 342 into the recess and conform it thereto. Thereafter, the core 10 could be placed into the recess 310 and cool air blown onto the platen 294, first film 342 and core, to mold the first film 342 into conformity with the core 10. The second film 348 would then be placed onto the platen, on top of the core 10, and warm air blown onto the second film 348 to soften it to a formable state. Another platen would then be moved into contact with the second film 348, pressing the second film 348 against the core 10 and the first platen 294, thereby, conforming the second film 348 to the contour of the core 10. Cool air would then be blown onto the second film 348, thereby molding the second film 348 onto the caplet 10. Lastly, the raised cutting edges of the recesses may cut through both of the first and second films 342, 348, thereby releasing enrobed products.

It is noted that the hot and cold air temperature ranges, as well as the vacuum pressure ranges, are within the knowledge of those skilled in the art.

In embodiments in which the shell is applied to the core by spraying, dipping, enrobing, or molding, the shell is preferably applied to the core in the form of a flowable material. The flowable material may comprise a dissolved, dispersed, or molten component, and optionally a solvent or fluid carrier component that may optionally be removed during processing, for example by drying. Regardless of the method by which it is applied, the finished shell, and accordingly the flowable material for making the shell, preferably comprises a film former. Optionally, the shell or readily soluble shell portion of the present invention may further comprise one or more thickeners, and various adjuvants and/or excipients as known in the art.

Any film former known in the art is suitable for use in the flowable material. Examples of suitable film formers include, but are not limited to, film-forming water soluble polymers, film-forming proteins, film-forming water insoluble polymers, and film-forming pH-dependent polymers. In one embodiment, the film former may be selected from cellulose acetate, ammonia methacrylate copolymer type B, shellac, hydroxypropylmethylcellulose, and polyethylene oxide, and combinations thereof.

Suitable film-forming water soluble polymers include water soluble vinyl polymers such as polyvinyl alcohol (PVA); water soluble polycarbohydrates such as hydroxypropyl starch, hydroxyethyl starch, pullulan, methylethyl starch, carboxymethyl starch, pre-gelatinized starches, and film-forming modified starches; water swellable cellulose derivatives such as hydroxypropyl cellulose (HPC), hydroxypropylmethyl cellulose (HPMC), methyl cellulose (MC), hydroxyethyl methylcellulose (HEMC), hydroxybutyl methylcellulose (HBMC), hydroxyethyl ethylcellulose (HEEC), and hydroxyethyl hydroxypropylmethyl cellulose (HEMPMC); water soluble copolymers such as methacrylic acid and methacrylate ester copolymers, polyvinyl alcohol and polyethylene glycol copolymers, polyethylene oxide and polyvinylpyrrolidone copolymers; and derivatives and combinations thereof.

Suitable film-forming proteins may be natural or chemically modified, and include gelatin, whey protein, myofibrillar proteins, coagulatable proteins such as albumin, casein, caseinates and casein isolates, soy protein and soy protein isolates, zein; and polymers, derivatives and mixtures thereof.

Suitable film-forming water insoluble polymers, include for example ethylcellulose, polyvinyl alcohols, polyvinyl acetate, polycaprolactones, cellulose acetate and its derivatives, acrylates, methacrylates, acrylic acid copolymers; and the like and derivatives, copolymers, and combinations thereof.

Suitable film-forming pH-dependent polymers include enteric cellulose derivatives, such as for example hydroxypropyl methylcellulose phthalate, hydroxypropyl methylcellulose acetate succinate, cellulose acetate phthalate; natural resins, such as shellac and zein; enteric acetate derivatives such as for example polyvinyl acetate phthalate, cellulose acetate phthalate, acetaldehyde dimethylcellulose acetate; and enteric acrylate derivatives such as for example polymethacrylate-based polymers such as poly(methacrylic acid, methyl methacrylate) 1:2, which is commercially available from Rohm Pharma GmbH under the tradename, EUDRAGIT S, and poly(methacrylic acid, methyl methacrylate) 1:1, which is commercially available from Rohm Pharma GmbH under the tradename, EUDRAGIT L, and the like, and derivatives, salts, copolymers, and combinations thereof.

One suitable hydroxypropylmethylcellulose compound for use as a thermoplastic film-forming water soluble polymer is "HPMC 2910", which is a cellulose ether having a degree of substitution of about 1.9 and a hydroxypropyl molar substitution of 0.23, and containing, based upon the total weight of the compound, from about 29% to about 30% methoxyl groups and from about 7% to about 12% hydroxypropyl groups. HPMC 2910 is commercially available from the Dow Chemical Company under the tradename METHOCEL E. METHOCEL E5, which is one grade of HPMC-291 0 suitable for use in the present invention, has a viscosity of about 4 to 6 cps (4 to 6 millipascal-seconds) at 20°C in a 2% aqueous solution as determined by a Ubbelohde viscometer. Similarly, METHOCEL E6 , which is another grade of HPMC-291 0 suitable for use in the present invention, has a viscosity of about 5 to 7 cps (5 to 7 millipascal-seconds) at 20°C in a 2% aqueous solution as determined by a Ubbelohde viscometer. METHOCEL E15, which is another grade of HPMC-2910 suitable for use in the present invention, has a viscosity of about 15000 cps (15 millipascal-seconds) at 20°C in a 2% aqueous solution as determined by a Ubbelohde viscometer. As used herein, "degree of substitution" means the average number of substituent groups attached to an anhydroglucose ring, and "hydroxypropyl molar substitution" means the number of moles of hydroxypropyl per mole anhydroglucose.

One suitable polyvinyl alcohol and polyethylene glycol copolymer is commercially available from BASF Corporation under the tradename KOLLICOAT IR.

As used herein, "modified starches" include starches that have been modified by crosslinking, chemically modified for improved stability or optimized performance, or physically modified for improved solubility properties or optimized performance. Examples of chemically-modified starches are well known in the art and typically include those starches that have been chemically treated to cause replacement of some of its hydroxyl groups with either ester or ether groups. Crosslinking, as used herein, may occur in modified starches when two hydroxyl groups on neighboring starch molecules are chemically linked. As used herein, "pre-gelatinized starches" or "instantized starches" refers to modified starches that have been pre-wetted, then dried to enhance their cold-water solubility. Suitable modified starches are commercially available from several suppliers such as, for example, A.E. Staley Manufacturing Company, and National Starch & Chemical Company. One suitable film forming modified starch includes the pre-gelatinized waxy maize derivative starches that are commercially available from National Starch & Chemical Company under the tradenames PURITY GUM and FILMSET, and derivatives, copolymers, and mixtures thereof. Such waxy maize starches typically contain, based upon the total weight of the starch, from about 0 percent to about 18 percent of amylose and from about 100% to about 88% of amylopectin.

Other suitable film forming modified starches include the hydroxypropylated starches, in which some of the hydroxyl groups of the starch have been etherified with hydroxypropyl groups, usually via treatment with propylene oxide. One example of a suitable hydroxypropyl starch that possesses film-forming properties is available from Grain Processing Company under the tradename, PURE-COTE B790.

Suitable tapioca dextrins for use as film formers include those available from National Starch & Chemical Company under the tradenames CRYSTAL GUM or K-4484, and derivatives thereof such as modified food starch derived from tapioca, which is available from National Starch and Chemical under the tradename PURITY GUM 40, and copolymers and mixtures thereof.

Any thickener known in the art is suitable for use in the flowable material of the present invention. Examples of such thickeners include but are not limited to hydrocolloids (also referred to herein as gelling polymers), clays, gelling starches, and crystallizable carbohydrates, and derivatives, copolymers and mixtures thereof.

Examples of suitable hydrocolloids (also referred to herein as gelling polymers) such as alginates, agar, guar gum, locust bean, carrageenan, tara, gum arabic, tragacanth, pectin, xanthan, gellan, maltodextrin, galactomannan, pusstulan, laminarin, scleroglucan, gum arabic, inulin, pectin, whelan, rhamsan, zooglan, methylan, chitin, cyclodextrin, chitosan. Examples of suitable clays include smectites such as bentonite, kaolin, and laponite; magnesium trisilicate, magnesium aluminum silicate, and the like, and derivatives and mixtures thereof. Examples of suitable gelling starches include acid hydrolyzed starches, and derivatives and mixtures thereof. Additional suitable thickening hydrocolloids include low-moisture polymer solutions such as mixtures of gelatin and other hydrocolloids at water contents up to about 30%, such as for example those used to make "gummi" confection forms.

Additional suitable thickeners include crystallizable carbohydrates, and the like, and derivatives and combinations thereof. Suitable crystallizable carbohydrates include the monosaccharides and the oligosaccharides. Of the monosaccharides, the aldohexoses e.g., the D and L isomers of allose, altrose, glucose, mannose, gulose, idose, galactose, talose, and the ketohexoses e.g., the D and L isomers of fructose and sorbose along with their hydrogenated analogs: e.g., glucitol (sorbitol), and mannitol are preferred. Of the oligosaccharides, the 1,2-disaccharides sucrose and trehalose, the 1,4-disaccharides maltose, lactose, and cellobiose, and the 1,6-disaccharides gentiobiose and melibiose, as well as the trisaccharide raffinose are preferred along with the isomerized form of sucrose known as isomaltulose and its hydrogenated analog isomalt. Other hydrogenated forms of reducing disaccharides (such as maltose and lactose), for example, maltitol and lactitol are also preferred. Additionally, the hydrogenated forms of the aldopentoses: e.g., D and L ribose, arabinose, xylose, and lyxose and the hydrogenated forms of the aldotetroses: e.g., D and L erythrose and threose are preferred and are exemplified by xylitol and erythritol, respectively.

In one embodiment of the invention, the flowable material comprises gelatin as a gelling polymer. Gelatin is a natural, thermogelling polymer. It is a tasteless and colorless mixture of derived proteins of the albuminous class, which is ordinarily soluble in warm water. Two types of gelatin - Type A and Type B - are commonly used. Type A gelatin is a derivative of acid-treated raw materials. Type B gelatin is a derivative of alkali-treated raw materials. The moisture content of gelatin, as well as its Bloom strength, composition and original gelatin processing conditions, determine its transition temperature between liquid and solid. Bloom is a standard measure of the strength of a gelatin gel, and is roughly correlated with molecular weight. Bloom is defined as the weight in grams required to move a half-inch diameter plastic plunger 4 mm into a 6.67% gelatin gel that has been held at 10°C for 17 hours. In a preferred embodiment, the flowable material is an aqueous solution comprising 20% 275 Bloom pork skin gelatin, 20% 250 Bloom Bone Gelatin, and approximately 60% water.

Suitable xanthan gums include those available from C.P. Kelco Company under the tradenames KELTROL 1000, XANTROL 180, or K9B310.

Suitable clays include smectites such as bentonite, kaolin, and laponite; magnesium trisilicate, magnesium aluminum silicate, and the like, and derivatives and mixtures thereof.

"Acid-hydrolyzed starch," as used herein, is one type of modified starch that results from treating a starch suspension with dilute acid at a temperature below the gelatinization point of the starch. During the acid hydrolysis, the granular form of the starch is maintained in the starch suspension, and the hydrolysis reaction is ended by neutralization, filtration and drying once the desired degree of hydrolysis is reached. As a result, the average molecular size of the starch polymers is reduced. Acid-hydrolyzed starches (also known as "thin boiling starches") tend to have a much lower hot viscosity than the same native starch as well as a strong tendency to gel when cooled.

"Gelling starches," as used herein, include those starches that, when combined with water and heated to a temperature sufficient to form a solution, thereafter form a gel upon cooling to a temperature below the gelation point of the starch. Examples of gelling starches include, but are not limited to, acid hydrolyzed starches such as that available from Grain Processing Corporation under the tradename PURE-SET B950; hydroxypropyl distarch phosphate such as that available from Grain Processing Corporation under the tradename, PURE-GEL B990, and mixtures thereof.

Suitable low-melting hydrophobic materials include fats, fatty acid esters, phospholipids, and waxes. Examples of suitable fats include hydrogenated vegetable oils such as for example cocoa butter, hydrogenated palm kernel oil, hydrogenated cottonseed oil, hydrogenated sunflower oil, and hydrogenated soybean oil; and free fatty acids and their salts. Examples of suitable fatty acid esters include sucrose fatty acid esters, mono, di, and triglycerides, glyceryl behenate, glyceryl palmitostearate, glyceryl monostearate, glyceryl tristearate, glyceryl trilaurylate, glyceryl myristate, GlycoWax-932, lauroyl macrogol-32 glycerides, and stearoyl macrogol-32 glycerides. Examples of suitable phospholipids include phosphotidyl choline, phosphotidyl serene, phosphotidyl enositol, and phosphotidic acid. Examples of suitable waxes include camauba wax, spermaceti wax, beeswax, candelilla wax, shellac wax, microcrystalline wax, and paraffin wax; fat-containing mixtures such as chocolate; and the like.

Suitable non-crystallizable carbohydrates include non-crystallizable sugars such as polydextrose, and starch hydrolysates, e.g. glucose syrup, corn syrup, and high fructose corn syrup; and non-crystallizable sugar-alcohols such as maltitol syrup.

Suitable solvents for optional use as components of the flowable material include water; polar organic solvents such as methanol, ethanol, isopropanol, acetone, and the like; and non-polar organic solvents such as methylene chloride, and the like; and mixtures thereof.

The flowable material for making the shell by spraying, dipping, enrobing, or molding may optionally comprise adjuvants or excipients, which may comprise up to about 30% by weight of the flowable material. Examples of suitable adjuvants or excipients include plasticizers, detackifiers, humectants, surfactants, anti-foaming agents, colorants, flavorants, sweeteners, opacifiers, and the like. Suitable plasticizers for making the core, the shell, or a portion thereof, by molding include, but not be limited to polyethylene glycol; propylene glycol; glycerin; sorbitol; triethyl citrate; tributyl citrate; dibutyl sebecate; vegetable oils such as castor oil, rape oil, olive oil, and sesame oil; surfactants such as Polysorbates, sodium lauryl sulfates, and dioctyl-sodium sulfosuccinates; mono acetate of glycerol; diacetate of glycerol; triacetate of glycerol; natural gums; triacetin; acetyltributyl citrate; diethyloxalate; diethylmalate; diethyl fumarate; diethylmalonate; dioctylphthalate; dibutylsuccinate; glyceroltributyrate; hydrogenated castor oil; fatty acids; substituted triglycerides and glycerides; and the like and/or mixtures thereof. In one embodiment, the plasticizer is triethyl citrate. In certain embodiments, the shell is substantially free of plasticizers, i.e. contains less than about 1%, say less than about 0.01% of plasticizers.

The openings may be made in the shell in any manner. A variety of methods of creating openings or holes in coatings are known in the pharmaceutical and other arts, and any of these may be used. As will be appreciated by one skilled in the art, different methods of making the openings may lend themselves to combination with different methods of manufacturing the dosage form and/or applying the shell thereto. The method of making the openings also depends on the nature and composition of the shell, and the outer coating if present. The openings may be formed in the shell of the present invention at any of several different steps in the process, depending partly on the process used to apply the shell to the core. For embodiments in which the shell is applied to the core by enrobing, the openings may be formed during formation of the cast film, after formation of the cast film but prior to application of the film to the core, during application of the film to the core, or after application of the film to the core. For embodiments in which the shell is applied to the core by dipping, transfer, spraying, or molding, the openings may be formed in the shell material during application of the shell to the core, or after application of the shell to the core.

For example, ablative methods employing erosion, melting, or vaporization of the shell by water jet erosion, sand blasting, grinding, arc vaporization, dielectric breakdown, ion beam sputtering, ultrasonic abrasive machining, cavitating fluid jet, or laser evaporation may be used to make the openings. Mechanical processes such as punching, perforating, slitting, piercing, drilling, masking followed by dipping, or vacuum removal may be used to make the openings. Chemical means, such as acid reactions, base reactions, solvent washing, acid etching with masking, photo etching with masking, anisotropic wet chemical etching, isotropic wet chemical etching, hydrophobization whereby dots of a nonwetting material are printed for example on a roller, or a burned mask effect may be used to make the openings. Methods involving the application of heat, such as melting with a hot iron, laser machining, arc evaporation, ultrasonic melting or cavitation, microwave heating, high energy methods such as plasma methods, infrared selective polymer curing, or induction heating with ferrous additives may be used to make the openings. Finally, additive methods such as painting, dipping, solvent washing, enrobing of the core with a woven material, strands or ribbons, spray coating with a masking material and removing the masking material by solvent or hydrophobic incompatibility, spraying through a masking sheet, using ribbons of material, or building up threads or webs or mats in the shell may also be used to create openings.

In one embodiment of the invention, the shell is applied to the core by thermal cycle or zero cycle molding, as described above, and the openings are formed via one or more protrusions on the inside surface of at least one of the mold assemblies. Each protrusion, which is adjusted for shape and size as desired, masks a small location on an underlying core, leaving behind an opening in the shell at the site of the protrusion. The mold assemblies may comprise a plurality of protrusions to form a plurality of corresponding openings in the shell. The protrusions may be located on the inside surface of only one mold assembly, say the upper mold assembly, or located within only a portion, i.e., one quadrant of the inside surface of one mold assembly, as desired. Or the protrusions may be arranged to form patterns, symbols, words, and the like, in the shell. See Figures 17a and 17b.

In another embodiment of the invention, the shell is applied to the core by enrobing, either using visually homogeneous films as described in U.S. Patent Nos. 5,146,730 and 5,459,983, or films having visually distinct portions as described in commonly assigned, copending U.S. Application Serial Nos. 10/146471, filed May 15, 2002 and 10/146722, filed May 15, 2002. In either case, the cast films used for the enrobing step are perforated either before, during, or after the enrobing process.

In one embodiment, in which the shell is applied to the core by enrobing, the openings may be formed during formation of the cast film by the mechanical method of casting the openings into the film using a suitably shaped or textured casting surface, e.g. chilled film forming roll (exterior surface 36 of casting drum 34 in the 10/146471, filed May 15, 2002 application). Suitable textures include surfaces with protrusions tall enough that the poured film will not envelope the protrusions, thus leaving openings in the cooled film. Figure 7 depicts such a forming roll 1010 having a surface 1020 having a suitable texture that creates voids, slits, or open areas in the film. The films arrive at the dye rolls 1040 already containing openings.

In a second embodiment, in which the shell is applied to the core via enrobing, the openings may be mechanically pierced or punched in the cast film after it is formed, and prior to its application to the core. For example, Figures 8 and 9 depict perforating apparatuses 1030 located between the forming rolls 1010 and dye rolls 1040. The perforating apparatus pierces, or punches openings into the formed film. In another such embodiment, utilizing the vacuum forming method of applying the shell to the core via enrobing, the film is vacuum formed on a vacuum forming plate (i.e., a plurality of porous platens 294 as described in the May 15, 2002 application) into the cavity shapes for depositing the cores into the recess, then the openings are formed prior to inserting the cores. In one particular such embodiment, the shaped film with recesses is removed from the forming plate (i.e., the plurality of porous platens 294), and travels to a perforating apparatus. In another particular embodiment, the shaped film with recesses remains in contact with the forming plate (i.e., the plurality of porous platens 294), and openings are formed therein via suitable ablative methods prior to insertion of the cores. One particularly suitable ablative method for this application employs a laser to "burn" the openings into the formed film in the desired shape, size and pattern.

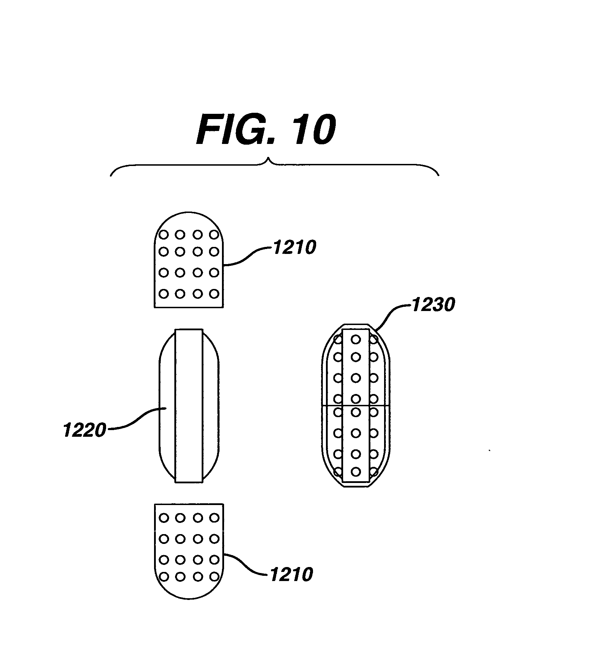

Another particular embodiment wherein openings are formed in the shell prior to its application to the core by enrobing is depicted in Figure 10. This method employs a capsule shell (comprising gelatin, starch, cellulose ethers, or other suitable materials as known in the art and described herein) to enrobe the core of the dosage form. The capsule shell is formed in two portions 1210 by dipping steel pins into a solution of shell material in fluid form as known in the art. The shell material is then solidified on the steel pins. Before removal of the solidified capsule shells from the forming pins, suitable means, such as for example a laser, is used to burn the openings in the capsule shell in the desired size, shape, and pattern. The cores 1220 are then enrobed with the capsule shells 1210 as known in the art, for example by shrink-fitting capsule shell halves onto compressed tablets as disclosed in U.S. Patent Nos. 5,415,868, 6,126,767, 5,464,631, 5,460,824, 5,317,849, 5,511,361, 5,609,010, 5,795,588 and 6,080,426, and PCT Appln. No. WO 97/37629 to produce a dosage form 1230 of the present invention.

In a third embodiment the openings may be formed in the shell during the application of the shell to the core. In such embodiments, the shell may be applied to the core by any suitable method, e.g. enrobing, dipping, transfer, spraying, or molding.

For example, in one embodiment wherein the shell is applied by enrobing, the surface of the rotary dies 1040 have a suitable texture that creates voids, slits, or open areas in the shell by perforating, piercing, or punching as the core is being enrobed with the cast film.

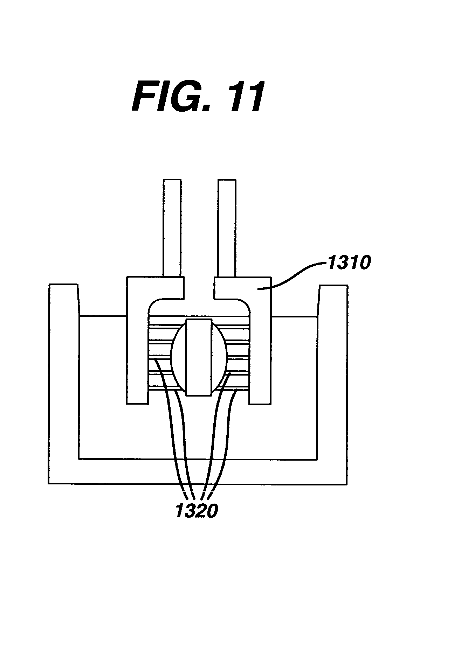

One example of a method of forming openings in the shell during application of the shell to the core by a dipping process is depicted in Figure 11. In this method, the core is held during dipping by a holder 1310 equipped with one or more, preferably a plurality of masking pins 1320 that hold the core, and mask the areas to be left uncovered by shell. The shape, size and arrangement of the masking pins 1320 determine the shape, size, and pattern of the openings in the shell.

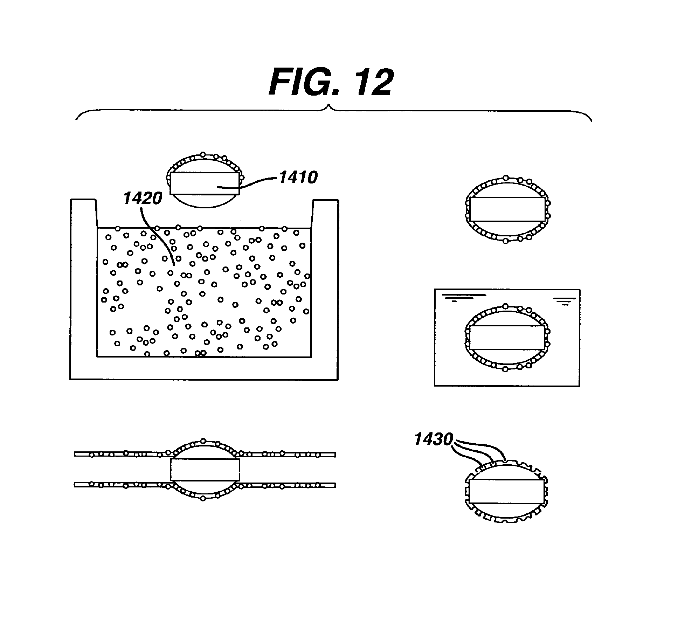

Another example of a method of forming openings in the shell during application of the shell to the core by a dipping process is depicted in Figure 12. Here the core is dipped into a foamed shell material 1420. The air bubbles in the foam will become openings 1430 in the finished solidified shell. In a similar embodiment, the openings are created during application of the shell by dipping the core into a suspension of solid wax non-pareils in film-forming shell material. The non-pareils are deposited onto the core along with the shell material. The shell material is then solidified. Next, heat is applied in order to melt the wax non-pareils, leaving openings in the shell in the size and shape of the wax non-pareils. In another embodiment, the core is dipped into a fluid material, e.g. polymer that reacts and solidifies upon activation by ultraviolet radiation, light, or heat. In this method, a highly specific or targeted energy source such as a laser is used to selectively solidify the portion of shell intended to remain on the tablet. After this treatment, the remaining fluid either drains off or is washed off. In a similar embodiment, the core surface may be coated with a powder, after which a laser can be used to selectively melt portions of the powder, leaving unmelted powder where openings are desired. The remaining material remains a powder and is shaken off.

One example of a preferred method of forming openings in the shell during application of the shell to the core by a transfer method similar to printing is depicted in Figure 13. A transfer plate 1510 surface has an engraved image with peaks and valleys. The shell material is applied in fluid form to the surface of the transfer plate 1510, and selectively fills in the valleys but does not cover the peaks. A "print pad" applicator 1520 picks up the shell material in the pattern of the image from the transfer plate 1510 and deposits, or transfers, the shell material in the desired pattern onto the surface of the core. Transfer plates offer the advantage of allowing indirect application of the pattern to the dosage form. Another suitable method of painting the shell material selectively onto the core is by powder coating, e.g. electrostatic deposition as disclosed in PCT Appln. No. WO 01/57144. Another suitable method of painting the shell material selectively onto the core is via ink jet printing as disclosed in.

In a fourth embodiment, the openings may be formed in the shell after the shell is applied to the core. One such embodiment is depicted in Figure 14. Here, the core 1610 has protrusions 1620, the shell 1630 is applied to surround the entire core, then the protrusions are ground off, e.g. by a pair of rotating grinding wheels 1640 to expose uncoated core (i.e. create openings 1650). In yet another example, the shell is applied to the core as multiple layers of a film, e.g. by enrobing. Areas of the film are then selectively burned off with a laser to create openings in the shell surface in the desired pattern and having any desired depth. In another such embodiment, a sintering process is used to deposit the shell. Powdered shell material is applied to the core surface, then heat is applied to partially, but not completely, fuse the powder particles, creating a porous shell surface.

Other preferred methods for forming openings in the shell after its application to the core employ mechanical methods such as punching, piercing, drilling, hot knife, and melting. One such mechanical method is depicted in Figure 15. Figure 15A depicts the use of hot pins or knives to melt and/or pierce the openings into the shell. Figure 15B depicts the drilling of openings in the shell. The melting or piercing tips depicted in Figure 15 may form the surface (or "business end") of either a punch mechanism, or a roller mechanism.

Another suitable mechanical method includes punching the openings into the formed shell as depicted in Figure 16. In this particular embodiment, the punched-out shell material remains in the cavity created by the punch, however a portion of the core surface becomes exposed as part of the interior of the cavity. Alternate methods for forming openings in the shell after its application to the core include ablative methods such as sand/grit blasting, grinding, arc vaporization, dielectric breakdown, ion beam sputtering, ultrasonic abrasive machining, cavitating fluid jet, laser evaporation; chemical methods such as selective application acid or base reaction, chemical photo etching; thermal methods such as melting using hot pins, laser machining, arc evaporation, ultrasonic melting or cavitation, microwave heating, and the like. These methods can optionally be employed to selectively remove shell material after first masking the area of shell intended to remain intact.

It will be appreciated that many additional methods, including but not limited to those in the broad categories of ablative, mechanical, chemical, thermal, or additive, though not specifically exemplified herein, are suitable for forming the openings in the shell of the present invention.

For example, after exiting the film casting apparatus but before being fed to the enrobing apparatus, the film sheets may be subjected to a mechanical process such as punching, perforating, slitting, piercing, or drilling to form one or more openings in the film sheets. For instance, slits may be formed in the cast or extruded film by ablative, mechanical, chemical, thermal, or additive methods.

The following non-limiting example further illustrates the invention.

### Example

### Part A: Preparation of compressed tablet core:

A compressed tablet core, comprising 500 mg of acetaminophen as the active ingredient, is prepared from the following components:

| Ingredient | Mg/Core |
|---|---|
| I. - Active and Excipients | |
| acetaminophen, USP | 500.0 |
| powdered cellulose, NF | 40.0 |
| pregelatinized starch, NF | 10.0 |
| sodium starch glycolate, NF | 10.0 |

| II. - Granulating Agent | |
|---|---|
| starch, NF | 40.0 |
| purified water, USP | q.s. |

| III - Dry Adds | |
|---|---|
| magnesium stearate, NF | 3.2 |
| Total | 603.2 |

The active and excipients of Part I are weighed in the proportions provided and added to a bowl of a fluid bed granulator such as an AEROMATIC brand granulator. The granulating agent (Part II) is prepared by adding the purified water to a processing tank with approximately 15 grams of water for each gram of starch NF. The starch is mixed in slowly, and the mixture is heated until the temperature reaches about 82 to 84° C. With the components of Part I in a heated fluidized state and an inlet air temperature of 75 to 85° C., the granulating agent is sprayed onto the powders. After all the granulating agent has been sprayed, the granulated powders are dried to a moisture content of about 1.4 to 1.9% as determined by loss on drying using for example a COMPUTRAC brand analyzer. The dried granulation is then sieved, for example, using a GLATT QUICK brand sieve stator No. 3, screen No. 1.5 mm, 1,000 RPM. The sieved and dried granulation is then blended with the powders of Part III using a suitable mixer such as a twin shell, ribbon or planetary mixer. The fmished blend is then loaded into the hopper of a rotary tableting machine and compressed into tablets using round deep concave tooling having a diameter of 7/16 inches. The average thickness of the resulting tablet cores is approximately 0.3 inches. The average hardness of the resulting tablet cores is approximately 10 Kp. The average tablet core weight is approximately 603.2 mg.

### Part B: Preparation of Shell Material as Cast Film for Enrobing:

A first gelatin based cast film for enrobing the core of part A, having a thickness in the range of approximately 0.02 inches, is prepared from the following components:

| | |
|---|---|
| Gelatin (150 Bloom) | 45% |
| Glycerin | 6% |
| Sorbitol | 2% |
| Water | 45% |
| Colorants | 2% |

The colorants are mixed with the water to form a homogenous solution. The dry gelatin granules are added to the colorant solution, and mixed for about 1 minute to completely wet the gelatin granules. The gelatin slurry is placed in a water bath and heated to 55°C to melt and dissolve the gelatin. The glycerin and sorbitol are then mixed in. The final solution is held at 55°C for approximately 10 hours to deaerate. The solution is then mixed at low speed until uniform (about 5 to 15 minutes), and transferred to a jacketed feed tank equipped with a propeller-type electric mixer. The gelatin solution is maintained at 55°C with continuous mixing during its use for forming the cast film for making the first shell portion of the present invention. The gelatin solution is then poured onto a casting drum (forming roll 1010), the surface of which is cooled to a temperature of approximately 25°C to form a cast film.

A second gelatin based cast film having similar thickness, but a second color, is prepared by the method described above.

The cooled first cast film is then fed between two rolls comprising a perforating apparatus (1030), which punches openings into the formed film. The openings are round, with a diameter of 750 microns, and are arranged in groups of 7, with one central opening encircled by 6 peripheral openings. The groups of 7 openings are spaced in the film at intervals corresponding to the cavities in the dye rolls 1040. The cooled second cast film is fed between two rolls with smooth surfaces, thus remaining continuous with no openings therein.

Next, the perforated first film and the non-perforated second film are fed between the dye rolls 1040, of an enrobing apparatus, together with the tablet cores of part A. The first and second films are pressed in contact with the tablet core, and with one another at an interface forming a seem around the belly band of the compressed tablet core. The resulting dosage form has a first shell portion of a first color, having openings therein, and a second shell portion of a second color, having substantially no openings therein.

### Porosity Example

Comparative samples were made of compressed tablets made according to the inventive processes described herein and non-inventive capsule plugs that could be used in gelatin shells. The samples were tested in a mercury porosimeter in the manner described above to determine their porosity. The results of a 10-sample comparison are shown below.

| Inventive % Porosity | Comparative % Porosity |
|---|---|
| 27.77 | 59.95 |
| 27.57 | 58.12 |
| 26.62 | 54.17 |
| 27.96 | 50.23 |
| 28.86 | 53.8 |
| 27.64 | 57.25 |
| 26.94 | 55.32 |
| 26.68 | 55.57 |
| 26.66 | 59.79 |
| | |
| 27.37 | 54.19 |

## Claims

1. A dosage form containing at least one active ingredient, which comprises a core and a shell surrounding at least a portion of the core, wherein the core has a density of at least about 0.9 g/cc and a percent porosity of less than 40%, the shell comprises one or more openings, the shell is readily soluble in gastrointestinal fluids, and the dosage form provides for immediate release of at least one active ingredient upon contact of the dosage form with a liquid medium.

2. The dosage form of claim 1, wherein the shell comprises a plurality of openings.

3. The dosage form of claim 1, wherein one or more openings contact the core.

4. The dosage form of claim 2, wherein at least a portion of the plurality of openings expose the core.

5. The dosage form of claim 1, wherein the core comprises a compressed tablet.

6. The dosage form of claim 5, wherein the compressed tablet has a hardness from about 2 to about 30 kp/cm^{2.}

7. The dosage form of claim 1, wherein the shell is applied to the core by dipping.

8. The dosage form of claim 1, wherein the shell is applied to the core by molding.

9. The dosage form of claim 1, wherein the shell is applied to the core by enrobing.

10. The dosage form of claim 1, wherein the dosage form provides for immediate release of at least one active ingredient contained in the core upon contact of the dosage form with a liquid medium.

11. The dosage form of claim 1, wherein the shell comprises gelatin.

12. The dosage form of claim 1, wherein the average shell thickness is in the range from about 100 to about 400 microns.

13. The dosage form of claim 1, wherein the shell comprises less than about 50% crystallizable carbohydrate.

14. The dosage form of claim 1, wherein the dosage form is substantially free of charge control agents.

15. The dosage form of claim 1, wherein a subcoating substantially surrounds the core.

16. The dosage form of claim 15, wherein the first and second shell portions are in direct contact with said subcoating.

17. The dosage form of claim 1, wherein the diameter or width of one or more openings is from about 200 to about 2000 microns.

18. The dosage form of claim 2, wherein said plurality of openings are arranged in a pattern such that they function as perforations, to separate a patch of the shell from the dosage form after contact of the dosage form with a suitable dissolution medium.

19. The dosage form of claim 18, wherein the separation of the patch exposes at least about 30% of the surface area of a major face on said dosage form to the dissolution medium.

20. The dosage form of claim 18, wherein the plurality of openings is in a circular pattern with openings having more than one size and/or shape.
